# EUROPEAN PATENT SPECIFICATION

(11) **EP 1 969 137 B1**
(45) Date of publication and mention of the grant of the patent: **05.10.2011**
(21) Application number: 05813533.6
(22) Date of filing: 22.11.2005
(51) Int. Cl.: C12Q 1/68

(54) **MULTIPLEX NUCLEIC ACID DETECTION**
MULTIPLEX-NUKLEINSÄURENACHWEIS
DETECTION MULTIPLEXE D'ACIDES NUCLEIQUES

(43) Date of publication of application: 17.09.2008
(73) Proprietor: Stichting Dienst Landbouwkundig Onderzoek, 6701 BH Wageningen (NL)
(72) Inventor: SCHOEN, Cornelis Dirk, NL-1751 GJ Schagerbrug (NL); SZEMES, Marianna, Bristol Bristol BS8 1TD (GB); BONANTS, Petrus Johannes Maria, NL-3911 BA Rhenen (NL)
(74) Representative: Hatzmann, Martin
(86) International application number: PCT/NL2005/000807
(87) International publication number: WO 2007/061284

(56) References cited:
- WO-A-00/36141
- WO-A-03/052142
- US-A1- 2005 026 180

## Description

The invention relates to multiplex detection of nucleic acid targets, more specifically detection on micro-arrays with tagged probes, even more specifically where such probes are padlock probes.

The accurate identification and detection of (pathogenic) micro-organisms or other targets of interest has become increasingly important in clinical diagnostics and pest management strategies. Traditionally, the predominant techniques used to identify pathogens have relied upon culture-based morphological approaches. These classical tools have been complemented in recent years by various culture-independent molecular characterizations, especially those involving PCR amplification of pathogen-specific nucleic acid targets (Atkins, S.D. and Clark, I.M. (2004) Fungal molecular diagnostics: a mini review. J. Appl. Genet., 45, 3-15; Lopez, M.M., Bertolini, E., Olmos, A., Caruso, P., Gorris, M.T., Llop, P., Penyalver, R. and Cambra, M. (2003) Innovative tools for detection of plant pathogenic viruses and bacteria. Int. Microbiol., 6, 233-243.). Although these approaches are generally effective, they target only a single pathogen per assay, making comprehensive screening of samples laborious and time-consuming.

To increase efficiency, it is desirable to develop multiplex assays, which can detect several targets simultaneously. Microarrays may enable highly parallel detection of diverse organisms (Bodrossy, L. and Sessitsch, A. (2004) Oligonucleotide microarrays in microbial diagnostics. Curr. Opin. Microbiol., 7, 245-254.). Typically, multiplex strategies involve either amplification with generic primers that target a genomic region containing species-specific information or multiple primer sets. Although such strategies are a marked improvement over traditional PCR-based assays, there are still serious limitations. Targeting a conserved genome region limits the analysis to a taxonomically defined group of pathogens, while combining several primer sets may present a significant technical challenge. Recently, universal amplification coupled with microarray analysis was suggested as an unbiased approach to pathogen detection (Vora, G.J., Meador, C.E., Stenger, D.A. and Andreadis, J.D. (2004) Nucleic acid amplification strategies for DNA microarray-based pathogen detection. Appl. Environ. Microbiol., 70, 3047-3054.). Although it overcomes the above-mentioned problems, the sensitivity at present is not sufficient for diagnostic purposes.

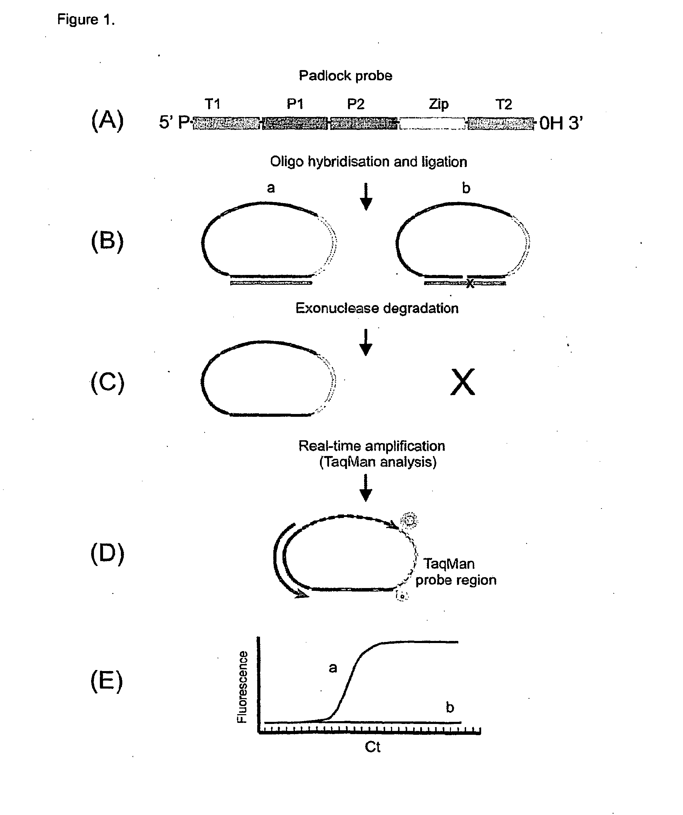

Padlock probes (PLP) are circularising probes which can offer a means of combining specific molecular recognition and universal amplification (or specific amplification and general recognition), thereby increasing sensitivity and multiplexing capabilities without limiting the range of potential target organisms. PLPs are long oligonucleotides of approximately 100 bases, containing target complementary regions at both their 5' and 3' ends (Fig. 1). These regions recognise adjacent sequences on the target DNA (Nilsson, M., Malmgren, H., Samiotaki, M., Kwiatkowski, M., Chowdhary, B.P. and Landegren, U. (1994) Padlock probes: circularizing oligonucleotides for localized DNA detection. Science, 265, 2085-2088.), and between these segments lie universal primer sites and a unique sequence identifier, the so-called ZipCode. Upon hybridisation, the ends of the probes get into adjacent position, and can be joined by enzymatic ligation converting the probe into a circular molecule that is threaded on the target strand. This ligation, and the resulting circular molecule can only take place when both end segments recognise their target sequences correctly. Non-circularised probes are removed by exonuclease treatment, while the circularised ones may be amplified with universal primers. This mechanism ensures reaction specificity, even in a complex nucleotide extract with a large number of padlock probes. Subsequently, the target-specific products are detected by a universal cZipCode microarray (Shoemaker, D.D., Lashkari, D.A., Morris, D., Mittmann, M. and Davis, R.W. (1996) Quantitative phenotypic analysis of yeast deletion mutants using a highly parallel molecular bar-coding strategy. Nat. Genet., 14, 450-456.). PLPs have been shown to have good specificity and very high multiplexing capabilities in genotyping assays (Hardenbol, P., Baner, J., Jain, M., Nilsson, M., Namsaraev, E.A., Karlin-Neumann, G.A., Fakhrai-Rad, H., Ronaghi, M., Willis, T.D., Landegren, U. and Davis, R.W. (2003) Multiplexed genotyping with sequence-tagged molecular inversion probes. Nat. Biotechnol., 21, 673-678.).

WO 03/052142 describes a padlock nucleotide probe comprising from 5' to 3' a first targeting domain, a reverse primer binding site for a selective primer, a blocking section comprising a restriction endonuclease cleavage site, a forward primer binding site, a tag and a second targeting domain.

US 2005/026180 describes a padlock nucleotide probe comprising from 5' to 3' a first targeting domain, a universal reverse primer, a cleavage site, a universal forward primer, a barcode and a second targeting domain. WO 00/36141 describes the use of a biotinylated spacer oligonucleotide for separating ligated and non-ligated padlock probes before amplification. However, these padlock probes all require further biotinylated.primers or spacers in order to reduce background amplification of non ligated padlock probes during the consecutive PCR steps.

The inventors now have found an efficient and reliable multiplex amplification and detection system, which makes use of improved, specifically designed padlock probes.

The present invention gives a solution for one of the problems associated with the use of padlock probes (PLPs), especially in multiplex assays, which is background amplification of non ligated PLP's during the consecutive PCR step.

To solve this, the invention comprises a padlock oligonucleotide probe comprising (from 5' to 3'):
a) a target specific nucleotide sequence 1 (T1);
b) a generic reverse primer binding site;
c) a nucleotide sequence bearing desthio-biotine;
d) a linker of at least 12 nucleotides
e) a unique cleavable sequence;
f) a generic forward primer binding site;
g) a ZIP-code sequence; and
h) a target specific nucleotide sequence 2 (T2),
wherein the T1 and T2 sequences are designed to be complementary to adjacent nucleotide stretches on the same target in such a way that after hybridization (and ligation of the outer ends) the padlock probe forms a circular molecule. The nucleotide bearing said desthiobiotin moiety is preferably coupled to the poly-uracil sequence through a thymidine linker. The unique cleavable sequence is preferably a mono-nucleotide sequence, such as a poly-uracil sequence. The generic forward primer binding site can comprise a T7 RNA polymerase recognition site. Preferred is an embodiment, wherein the ZIP code sequence is the complementary strand of a nucleotide sequence on an array.

Another aspect of the present invention is a padlock nucleotide probe comprising from 5' to 3'
a) a target specific nucleotide sequence 1 (T1);
b) a unique reverse primer binding site;
c) a nucleotide sequence bearing desthio-biotine;
d) a linker of at least 12 nucleotides
e) a unique cleavable sequence;
fe) a unique forward primer binding site; and
g) a target specific nucleotide sequence 2 (T2),
wherein the T1 and T2 sequences are designed to be complementary to adjacent nucleotide stretches on the same target in such a way that after hybridization (and ligation of the outer ends) the padlock probe forms a circular molecule. The unique cleavable sequence is preferably a mono-nucleotide sequence, such as a poly-uracil sequence. Optionally, the padlock nucleotide probe of this embodiment contains a universal ZIP code sequence, preferably between the unique forward primer binding site and the T2 sequence.

The invention also provides a method for the detection of a target nucleotide sequence comprising:
a. adding to a sample which contains said target, one or more padlock nucleotide probes of the invention, which have target specific sequences capable of hybridisation to said target;
b. allowing annealing of the padlock nucleotide probe to said target;
c. circularisation of padlock probe by ligation;
d. capturing the padlock nucleotide probes by bringing them into contact with a solid support coated with a second member of a binding pair
e. linearising the padlock nucleotide probe by cleaving the unique cleavable sequence;
f. washing of the beads to remove any unbound oligonucleotides
g. elution of the PLP probe from the solid support
h detection of the ZIP-code sequence.
In said method the detection of the ZIP-code preferably comprises the steps of:
i. amplification of the padlock nucleotide probe using the generic primers;
j. labelling the amplified padlock nucleotide probe;
k. testing for presence of the ZIP-code by hybridising said padlock nucleotide probe with at least one sequence which is capable of hybridisation with said ZIP-code sequence, wherein said hybridisation preferably takes place at a solid support, such as a (micro-) array.
Alternatively in said method the detection of the ZIP-code can be performed also directly:
i. testing for presence of the ZIP-code by hybridising said padlock nucleotide probe with at least one sequence which is capable of hybridisation with said ZIP-code sequence, wherein said hybridisation preferably takes place at a solid support, such as a (micro-) array or gold beads.
j. labelling of the hybridized padlock nucleotide probe on the solid support with a fluorescent probe directed against the first member of a binding pair of the padlock or with fluorescently labelled Biobarcode labelled gold beads.

These methods preferably comprise an NaOH denaturation step before capturing of the padlock nucleotide probes. Streptavidin will bind the desthiobiotin on the PLP. The elution of step g. is performed with biotin. If the unique cleavable sequence is a poly-uracil sequence, cleavage can preferably be effected by treatment with uracil-N-glycosidase and endonuclease IV.

In the case of BCA detection , the linearized PLP stays bound to the streptavidin and goldbeads bearing both ZIP codes complementary to the nucleotide sequence in the padlock and fluorescent barcode nucleotides, will be hybridized.

Another aspect of the invention is a method for the detection of a target nucleotide sequence comprising:
a. adding to a sample which contains said target, one or more padlock nucleotide probes, which have target specific sequences capable of hybridisation to said target;
b. allowing annealing of the padlock nucleotide probe to said target;
c. circularisation of padlock probe by ligation;
d. capturing the padlock nucleotide probe using a solid support coated with streptavidin;
e. linearising the padlock nucleotide probe by cleaving the unique cleavable sequence;
f. eluting the padlock nucleotide probe from the solid support,
g. amplifying the eluted padlock nucleotide probe using the unique primers;
h. monitoring and detecting amplification..
In said method, the amplification and monitoring of the amplification is preferably performed on an Open Array™ system (BioTrove). The method can optionally comprise an NaOH denaturation step before capturing of the padlock nucleotide probes.

Streptavidin will bind desthiobiotin on the PLP. The elution of step f. is performed with biotin.

If the unique cleavable sequence is a poly-uracil sequence, cleavage can preferably be effected by treatment with uracil-N-glycosidase and endonuclease IV.

A further aspect of the invention is the use of padlock nucleotide probes according to the invention for the multiplex detection of nucleotide sequences.

A next aspect of the invention is a test kit comprising multiple padlock probes according to the invention, wherein each padlock probe is designed to recognise a unique target.

### DESCRIPTION OF THE FIGURES

**Figure 1****.** Scheme of conventional PLP ligation and real-time PCR to quantify single mismatch discrimination. **(A)** PLPs contain target-complementary sequences at the 5' and 3' ends (T1, T2), flanking the universal primer sites (P1, P2) and the unique identifier ZipCode sequence (Zip). **(B)** T1 and T2 bind to adjacent sequences on the target, and in the case of a perfect match, the probe may be circularised by a ligase (a). Mismatch-containing molecules are expected to be discriminated, and no ligation should occur (b). **(C)** Unreacted probes are removed by exonuclease treatment. **(D)** Circularised probes are amplified using two universal primers and amplification is monitored in real-time using a TaqMan probe, which detects the ZIP-code. **(E)** Ligation yields with different target oligonucleotides can be accurately quantified based on the threshold cycle (Ct) values of amplification.

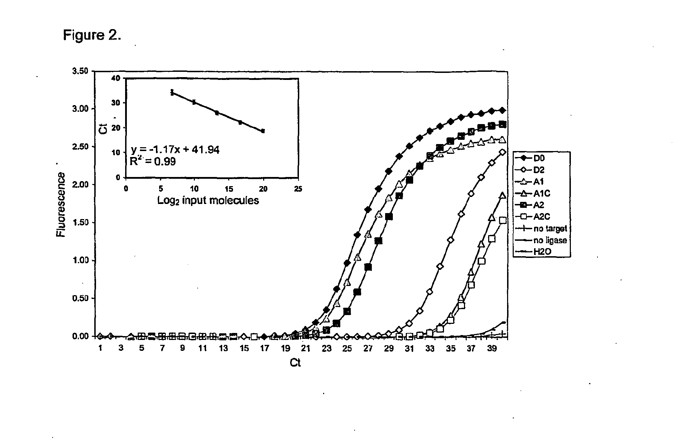

**Figure 2****.** Amplification curves of a representative experiment to optimize PLP design for mismatch discrimination. The respective ligation targets are indicated for each sample (Table 2A). In the insert the calibration curve of the real-time PCR is shown, which was used to determine amplification efficiency (E=0.81).

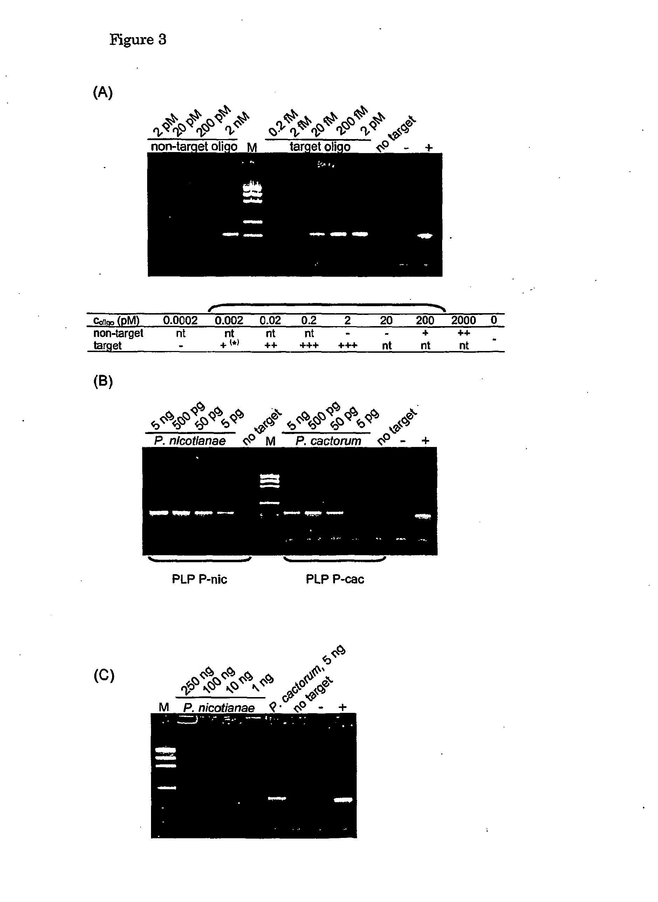

**Figure 3****.** Sensitivity and discriminatory range of diagnostic PLPs were assessed by using synthetic complementary oligonucleotides and genomic DNAs.
   **(A)** PLP P-inf was ligated on serial dilutions of oligonucleotides representing closely related, non-target and target sequences, respectively. Reactions were scored as either negative or positive, as shown in the table below ('nt' stands for 'not tested'). Detection threshold is indicated by an asterisk and the magnitude of discriminatory range is shown. **(B)** Detection of dilution series of *P. nicotianae* and *P. cactorum* genomic DNAs using the corresponding PLPs. Template DNAs are indicated above the picture, while the used PLPs are shown below. **(C)** Ligation of PLP P-cac on genomic DNA of *P. nicotianae* does not result in a positive signal even in the presence of very high amount of DNA. Amount of template DNAs are shown above the picture.
**Figure 4****.** Layout of multi-chamber universal tag array. Deposition scheme and sequences of cZipCode probes.
**Figure 5****:** Schematic approach of the traditional PLP technology for detection of pathogens (for details see text).

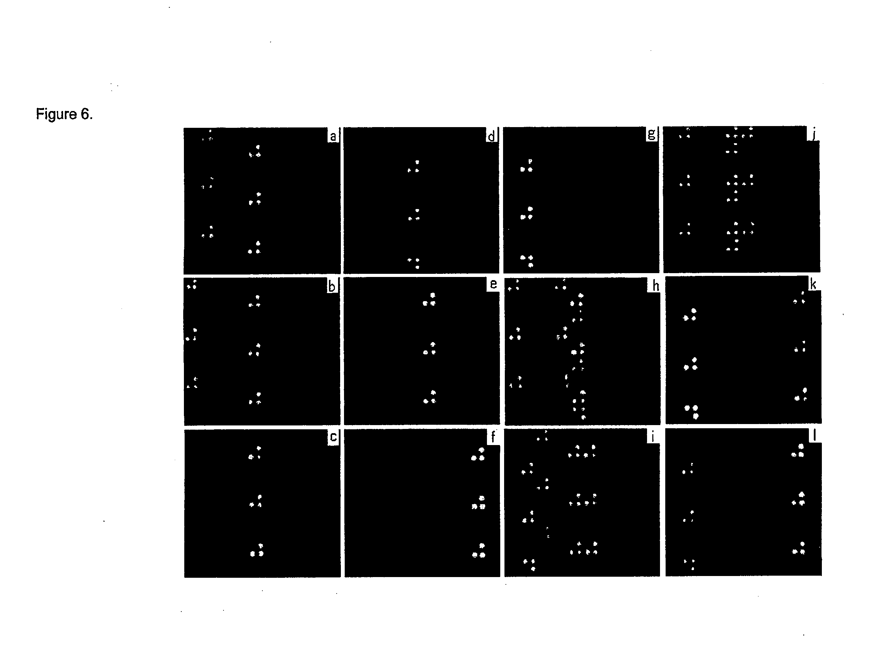

**Figure 6****.** Detection of genomic DNAs corresponding to individual (a - g) and complex pathogen samples (h-1) on a universal microarray. The analyzed targets were: (a) *P. cactorum;* 1 ng (b) *P. nicotianae,* 1 ng; (c) *P. sojae,* 1 ng; (d) *R. solani* AG 4-2, 1 ng; (e) *M. hapla,* 1 ng; (f) *F*. *oxysporum,* 1 ng; (g) *M roridum,* 1 ng; (h) *Pyt. ultimum,* 500 pg; *M hapla,* 500 pg and *P. nicotianae,* 500 pg; (i) *P. infestans,* 500 pg; *R. solani* AG 4-2, 500 pg and *M roridum,* 500 pg; (j) *P. cactorum,* 500 pg; *R. solani* AG 4-1, 500 pg and *V. dahliae,* 500 pg. (k) *F. oxysporum,* 0.5 pg and *M roridum,* 500 pg; (1) *F. oxysporum,* 500 pg and M *roridum* 5 pg;
**Figure 7****.** Three preferred probes of the invention, the standard Padlock probe, the PRI-lock probe and the LUNA-probe.
**Figure 8** Schematic approach of PLP technology with the newly designed standard PLPs for detection of pathogens (for details see text)
**Figure 9****.** Schematic overview PRI-lock probe based amplification combined with generic TaqMan detection (for details see text)

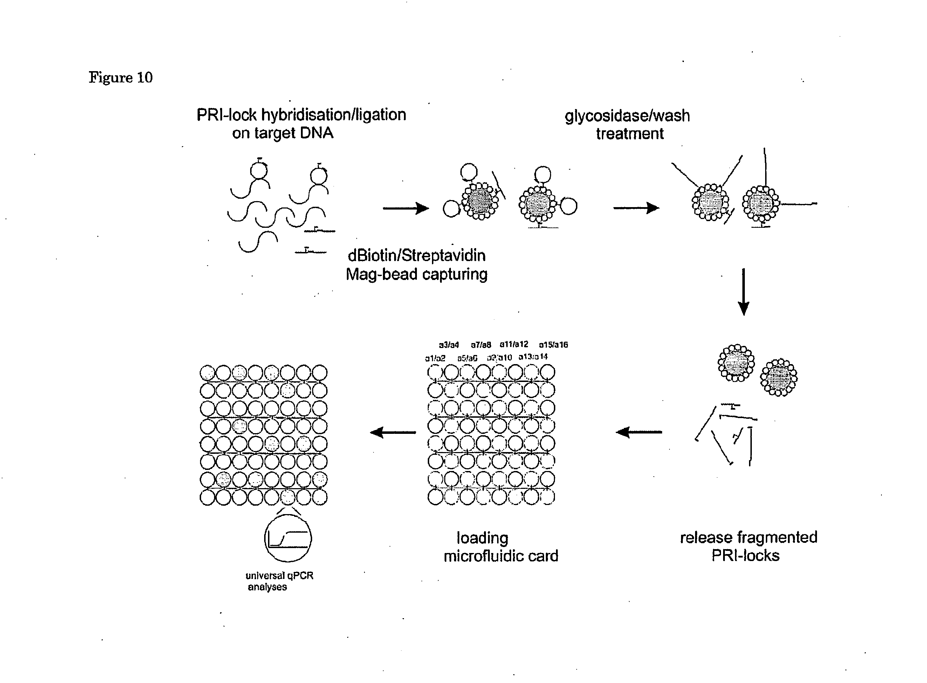

**Figure 10****.** Schematic overview of the PRI-lock probe based multiplex detection in combination with the Open Array™ system (BioTrove).

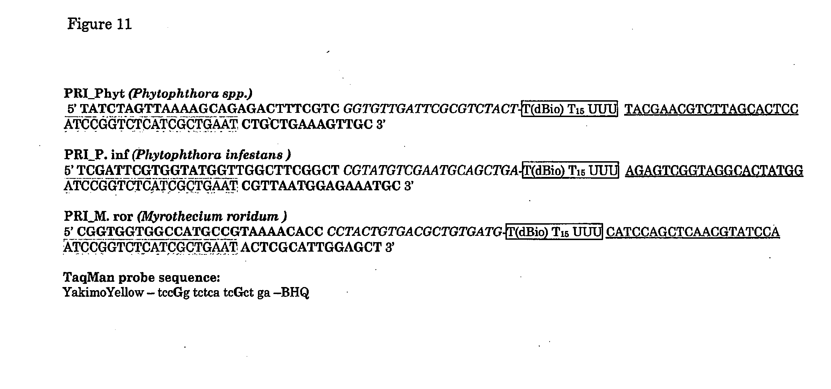

**Figure 11****.** Sequences of the designed PRI-lock probes and of the universal TaqMan probe. Different parts of the PRI-locks are indicated by different lettertypes. Bold : target complementary sites. Italic: reverse primer binding site. Underlined: forward primer binding site. Gray box: the universal TaqMan probe region. The deoxy-uracil cleavage site, the linker and the desthiobiotin moiety are indicated in open boxes. LNA (locked nucleic acid) nucleotides in the TaqMan probe sequence are shown in capitals
**Figure 12****.** Amplification plots of real-time PCR performed on ligated PRI-lock probes.
   The data points for PRI_M.ror, PRI_Phyt and PRI_P.infare shown in ○, ◇ and Δ, respectively.
**Figure 13****.** Calibration curves for target quantification using PRI-lock probe ligation and subsequent real-time PCR. The Ct values were plotted in function of log2 (input DNA concentration in the ligation, expressed as fg/µL). The data points and equations for PRI_M.ror, PRI_Phyt and PRI_P.inf are shown in ▲,◆ and ■respectively. Data points which were not in the linear range of quantification were omitted from calibration curve equations (open symbols).
**Figure 14****.** Scheme for array based and solution based multiplex detection with LUNA probes.
**Figure 15****.** Principle of NASBA reaction in combination with Molecular Beacon (AmpliDet RNA).
**Figure 16****.** Examples of two LUNA probes for the detection of *Verticillium dahliae* and *Phytopthora cactorum.,* the targets in NASBA the produced RNA amplicons and two specific molecular beacons for the produced RNA's
   Different parts of the LUNA probes are indicated by different lettertypes. Bold : target complementary sites. Small letters: reverse primer binding site. Under lined, Italic and in capital: T7 recognition site. Small letters and underlined: forward primer binding site. Gray box: the specific hybridization site. The deoxy-uracil cleavage site, the linker and the desthiobiotin moiety are indicated in open boxes.

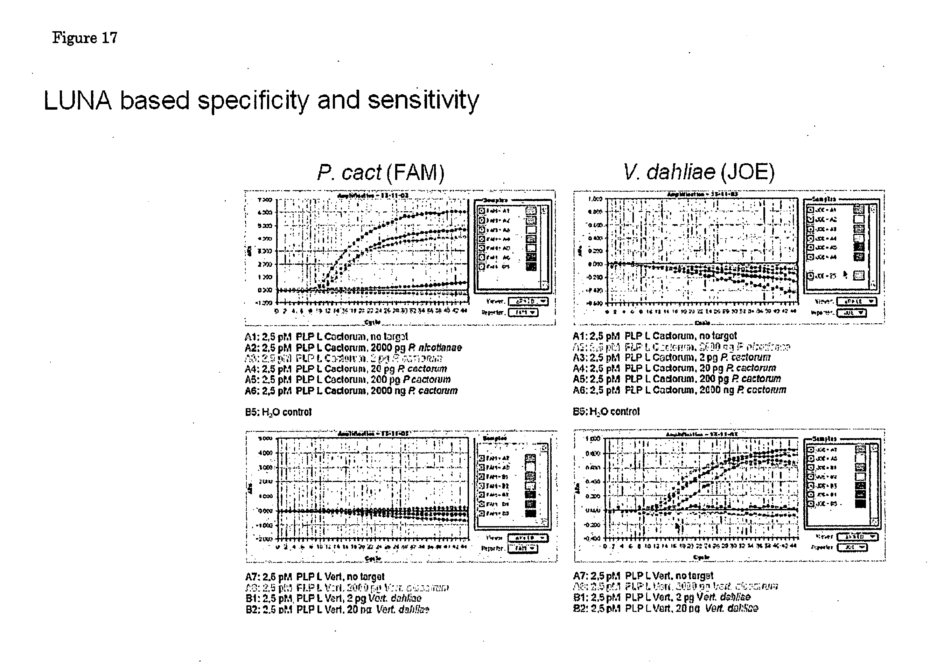

**Figure 17****.** Amplification plots of real-time NASBA and Molecular Beacon detection performed with two ligated LUNA probes showing specificity and sensitivity.

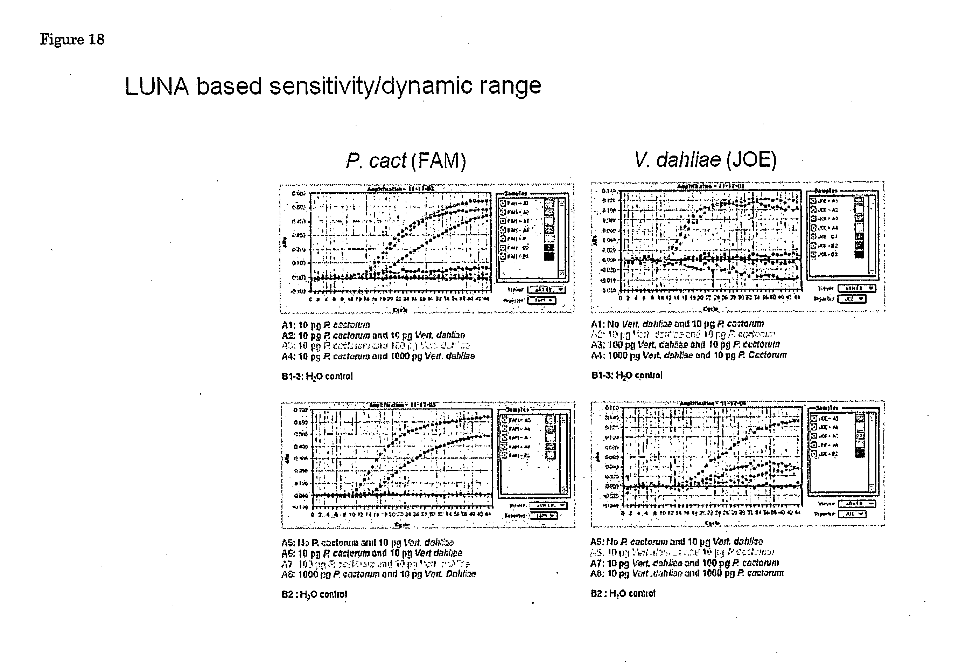

**Figure 18****.** Amplification plots of real-time NASBA and Molecular Beacon detection performed with two ligated LUNA probes showing sensitivity and dynamic range.

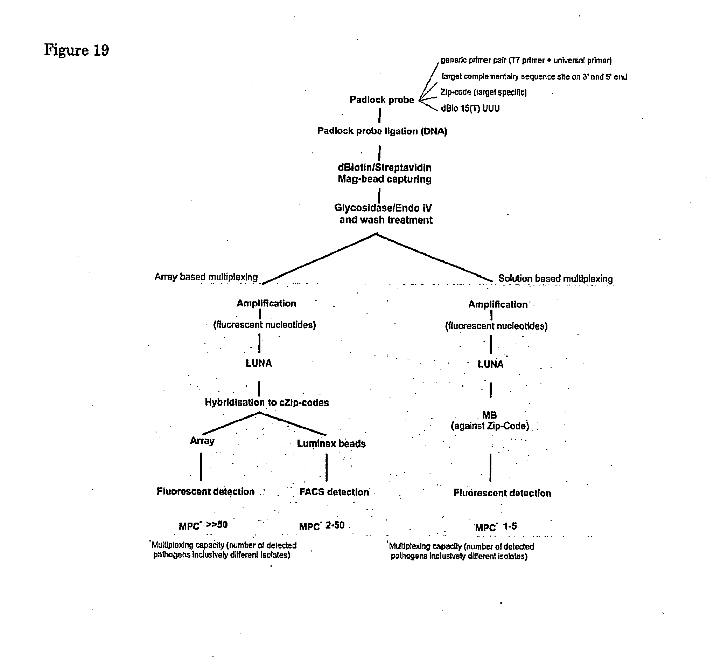

**Figure 19****.** Application scheme for the LUNA technology. After LUNA probe hybridization, ligation, exonuclease and glycosidase treatment, NASBA is performed. Visualization of the produced NASBA RNA amplicons can be performed on array, Luminex beads or with Molecular Beacons.

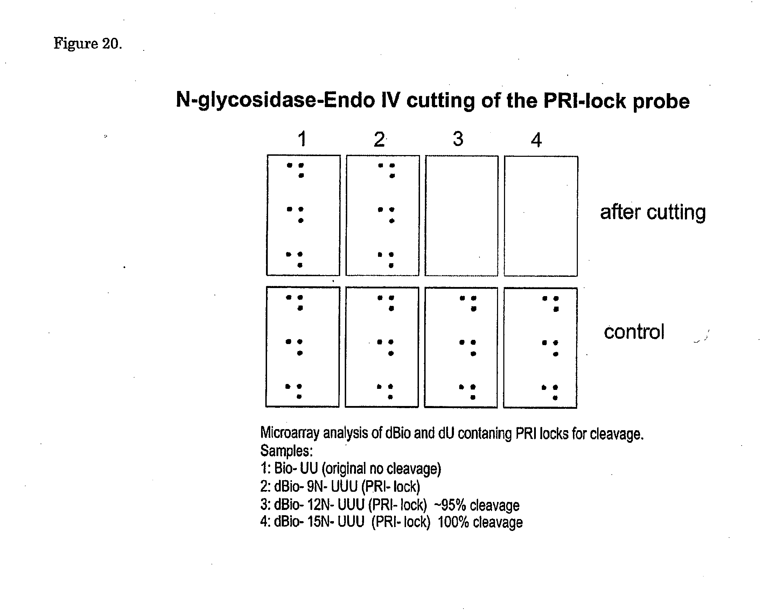

**Figure 20****.** N-glycosidase -Endo IV cutting of the PRI lock probes with different length of spacer.

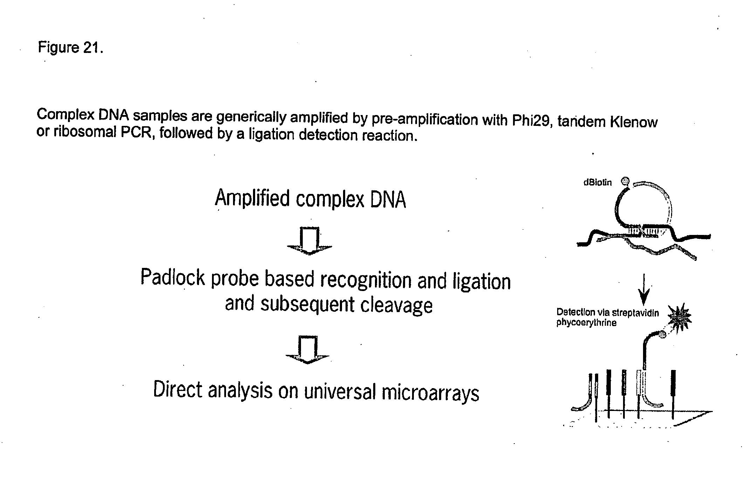

**Figure 21** Schematical view of complex DNA samples which are generically amplified by pre-amplification with Phi29, tandem Klenow or ribosomal PCR followed by a ligation detection reaction (LDR) using padlock probes.

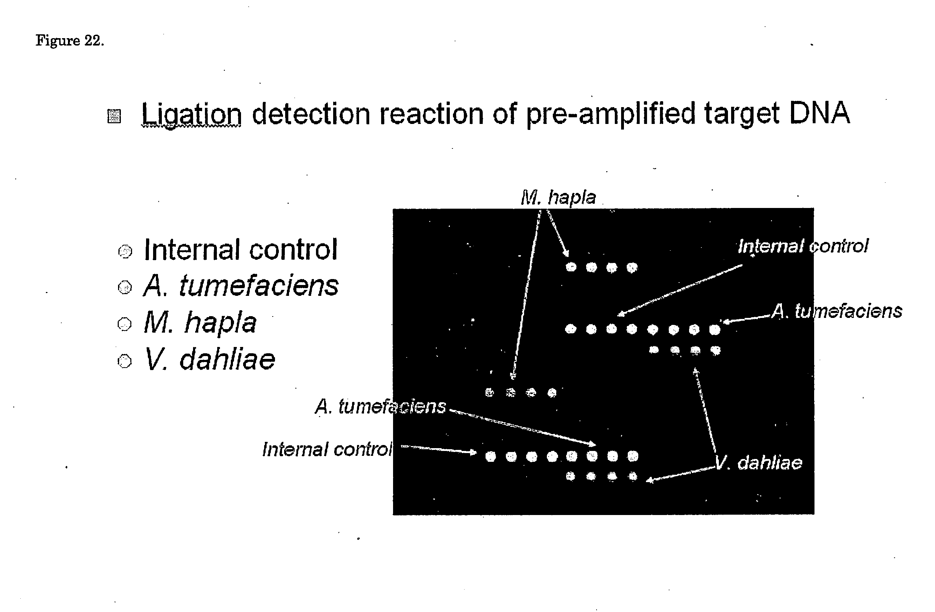

**Figure 22** Detection of ribosomal PCR preamplified genomic DNAs with the ligation detection reaction.. The analyzed targets were: *A. tumefaciens, M hapla* and *V. dahliae*

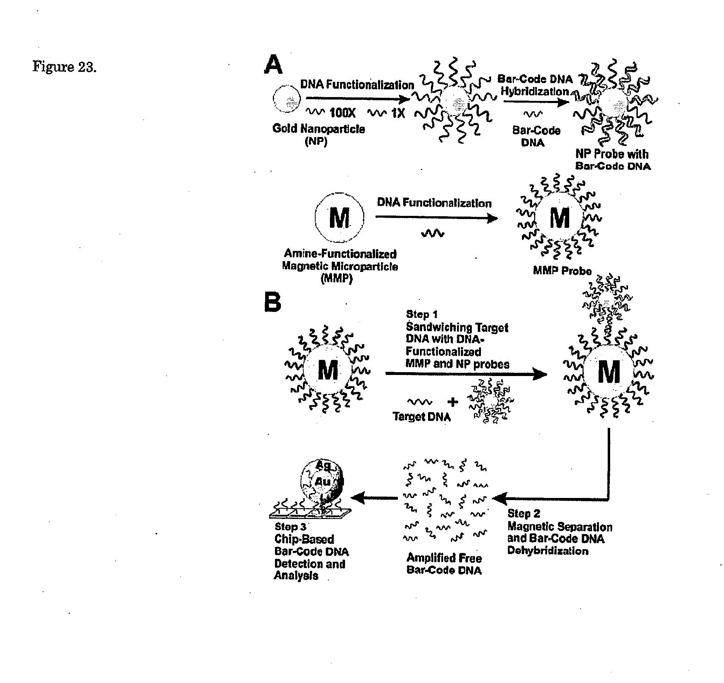

**Figure 23** DNA_BCA assay (A) Nano particle and magnetic microparticle probe preparation. (B) Nano particle-based PCR less DNA amplification scheme (according to Jwa-Min Nam, Savka I. Stoeva and Chad A. Mirkin. Bio-Bar-Code-Based DNA Detection with PCR-like Sensitivity. JACS, 126, 5932-5933 (2004)).

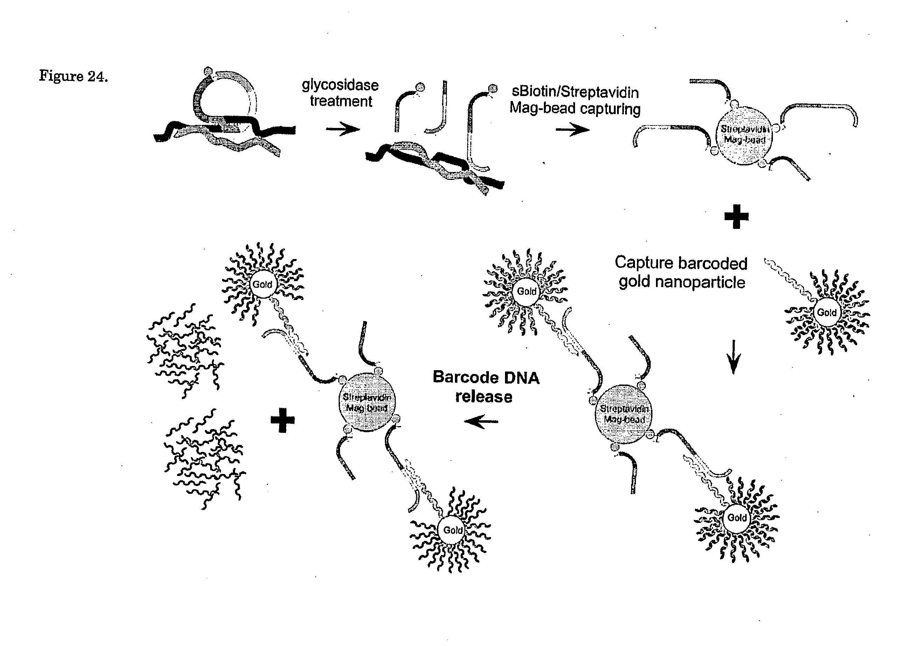

**Figure 24** Principle of Bio-barcode based signal amplification of a ligated standard PLP.

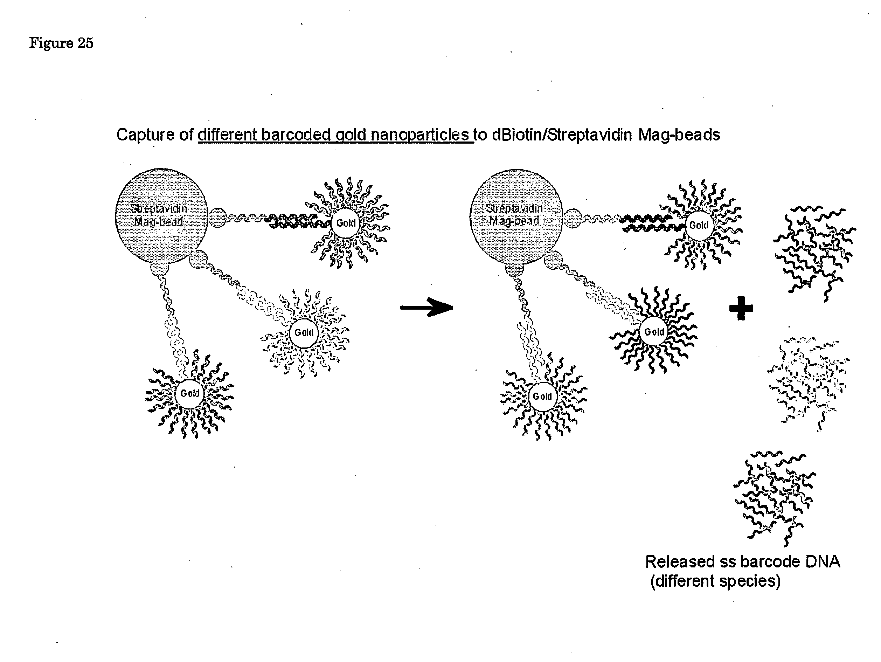

**Figure 25** Principle of Bio-barcode based multiplex signal amplification, after capturing of different ligated standard PLPs.

### DEFINITIONS

The term "hybrid" refers to a double-stranded nucleic acid molecule, or duplex, formed by hydrogen bonding between complementary nucleotides. The terms "hybridise" or "anneal" refer to the process by which single strands of nucleic acid sequences form double-helical segments through hydrogen bonding between complementary nucleotides.

The term "ligation" refers to the process of enzymatically joining two or more nucleotide sequence together by coupling the 5' P moiety of one nucleotide to the 3' OH moiety of a second nucleotide, thereby leaving the polynucleotide backbone intact, which thus will result in a concatenated, normal nucleotide sequence. The enzyme used for ligation is a ligase.

By "amplification" is meant the construction of multiple copies of a nucleic acid sequence or multiple copies complementary to the nucleic acid sequence using at least one of the nucleic acid sequences as a template. Methods of the invention can in principle be performed by using any nucleic acid amplification method, such as the Polymerase Chain Reaction (PCR; Mullis 1987, U.S. Pat. No. 4,683,195, 4,683,202, and 4,800,159) or by using amplification reactions such as Ligase Chain Reaction (LCR; Barany 1991, Proc. Natl. Acad. Sci. USA 88:189-193; EP Appl. No., 320,308), Self-Sustained Sequence Replication (3SR; Guatelli et al., 1990, Proc. Natl. Acad. Sci. USA 87:1874-1878), Strand Displacement Amplification (SDA; U.S. Pat. Nos. 5,270,184, and 5,455,166), Transcriptional Amplification System (TAS; Kwoh et al., Proc. Natl. Acad. Sci. USA 86:1173-1177), Q-Beta Replicase (Lizardi et al., 1988, Bio/Technology 6:1197), Rolling Circle Amplification (RCA; U.S. Pat. No. 5,871,921), Nucleic Acid Sequence Based Amplification (NASBA), Cleavage Fragment Length Polymorphism (U.S. Pat. No. 5,719,028), Isothermal and Chimeric Primer-initiated Amplification of Nucleic Acid (ICAN), Ramification-extension Amplification Method (RAM; U.S. Pat. Nos. 5,719,028 and 5,942,391) or other suitable methods for amplification of DNA. The product of amplification is termed an amplicon. Amplification as used in the present invention also comprises BioBarCode amplification (BCA) as described by Jwa-Min Nam, Savka I. Stoeva and Chad A. Mirkin. Bio-Bar-Code-Based DNA Detection with PCR-like Sensitivity. JACS, 126, 5932-5933 (2004). Although this is not an amplification in the sense that multiple copies of the original are made, it constitutes an amplification of the signal.

The term "primer" as used herein refers to an oligonucleotide which is capable of annealing to the amplification target (the "primer binding site") allowing a polymerase to attach thereby serving as a point of initiation of DNA or RNA synthesis when placed under conditions in which synthesis of primer extension product which is complementary to a nucleic acid strand is induced, i.e., in the presence of nucleotides and an agent for polymerization such as DNA polymerase and at a suitable temperature and pH. The (amplification) primer is preferably single stranded for maximum efficiency in amplification. Preferably, the primer is an oligodeoxy ribonucleotide. The primer must be sufficiently long to prime the synthesis of extension products in the presence of the agent for polymerization. The exact lengths of the primers will depend on many factors, including temperature and source of primer. Normally, primers come in sets including one forward and one reverse primer as commonly used in the art of DNA amplification such as in PCR amplification. Thus also the "primer binding sites" on the target DNA are present in a set of one for the forward and one for the reverse primer.

### DETAILED DESCRIPTION OF THE INVENTION

The present invention gives a solution for one of the problems associated with the use of padlock probes (PLPs), especially in multiplex assays, which is background amplification of non ligated PLP's during the consecutive PCR step. Padlock probes also have some tendency to linear dimer formation as a result of cross reactive ligation, the corresponding ligation products can easily be distinguished from circularized probes by exonucleolytic degradation. The exonuclease treatment reduces the number of such linear monomeric and dimeric molecules by almost three orders of magnitude with negligible effects on circularized probes.

Removal of unreacted probes further reduces ligation independent amplification events that may otherwise occur through accidental primers or templating of polymerization by the large number of linear probes. This can be effected by linearizing the exonuclease treated probes by cleavage at a unique site, which was introduced in the probe. It is paramount that the cleavage site is unique to prevent multiple occurrences in the probe or in the target nucleotides. Thus, it would be possible to use restriction sites which are recognized and cleaved by a restriction enzyme, but in that case the site should be basically non-existing in the sample nucleotide material and only occur once in each padlock probe. A few examples of such a sequence would be CTAAGNNNNNCTTAG (wherein N denotes any nucleotide), which is cleavable by the enzyme C EcoO109I; the sequence TGGCGACGAAAACCGCTTGGAAAGTGGCTG, which is cleavable by the enzyme F-TflI; ACCTACCATTAACGGAGTCAAAGGCCATTG, which is cleavable by the enzyme F-TflII, TAGGTACTGGACTTAAAATTCAGGTTTTGT, which is cleavable by the enzyme F-TflIII; CAAAACGTCGTAAGTTCCGGCGCG which is cleavable by the enzyme H-DreI; and GAGTAAGAGCCCGTAGTAATGACATGGC, which is cleavable by the enzyme I-BmoI.

However, all these enzymes only cut double-stranded DNA. To make the DNA double-stranded a complementary strand to the site that has been built in the padlock probe should be added, which then anneals to the padlock, forming a short double-stranded sequence. Addition of the restriction enzyme then will cut the padlock and linearize it.

Alternatively, Hardenbol et al. (*supra*) described a different solution for this problem for use in a single-stranded molecule by using uracil depurination with uracil-N-glycosidase and cleavage of the sugar backbone with a heat step. However, it was found that this does not lead to an efficient cleavage of the backbone, which thus still leaves contamination during the amplification reaction. Use of EndoIV nuclease according to this invention in stead of the heat step is much more efficient, thereby reducing background contamination and thus increasing the sensitivity of the assay.

Thus, in one embodiment, the PLP of the invention preferably contains a polyuracil site for enabling linearization of the probes. Preferably, the unique cleavage sequence is introduced just to the 5' side of the unique forward primer binding site, which, after cleavage becomes the most 5' part of the linearized molecule. In the same way, the unique reverse primer binding site should be positioned as close as possible 5' of the unique cleavage sequence, in order to leave, upon cleaving, said reverse primer binding site at the most 3'end of the linearized molecule.

In between the two universal primer binding sites then the ligated target recognition sites and the ZIP code will be present, which would ensure a proper amplification of the parts of the PLP that are used for giving a specific reaction in the assay.

In case of cleavage of the poly-uracil sequence a treatment with endonuclease IV is performed for efficient cutting the deoxy-ribose phosphate backbone at the ends of the linearized polynucleotide.

Further, linearization of the PLP before amplification ensures that PLPs which have not been ligated at the target site will not be amplified. They are also cleaved at the unique cleavage site, leaving one short piece of DNA with only the unique reverse primer binding site, and another, a bit longer stretch, bearing the unique forward primer binding site. Since those pieces are not joined anymore, an amplification step using the universal primer set will not be able to generate amplification products to these incomplete PLPs. Therefore, by linearizing the PLP, an increase in the detection limit is obtained, since the background (noise) amplification signal will be much lower.

While for specifically recognised restriction sites, the length of the site is fixed, the length of the poly-uracil sequence is not critical, as long as it gives a good cleavage upon application of uracil-N-glycosidase and endo IV nuclease. Preferably, the stretch of uracil nucleotides has at least 2 uracil bases. In principal, there is no upper limit to the length of the poly-uracil sequence, it will in practice be limited by the technical requirements of the synthesis.

Although also other restriction enzyme sites could be used to linearize the PLP, a poly-uracil site is preferred because this will normally not be present in any of the target nucleotides, nor in any of the further building blocks of the PLP. Thus, it provides an unique site, with little or no chance of disturbing other nucleotides which are present in the reaction of the assay. Further, use of the poly-uracil enables linearization of the padlock probe while it is still in the single-stranded state and thus, no additional mixing with complementary oligonucleotides is necessary. Also the used uracil-N-glycosidase and endonuclease IV have no negative effect on the other nucleotides in the reaction

The target molecules, which have to be assayed, can be any form of DNA or RNA, such as genomic DNA, cDNA, mitochondrial DNA, nuclear DNA, messenger RNA, ribosomal RNA and the like. The type of nucleotide is unimportant, but the target should be able of being specifically recognised by the corresponding padlock nucleotide probe.

Advantageously, the target nucleotides, which are present in the sample to be assayed, are randomly cut into smaller fragments of 100-1000 basepairs. This can be done using standard methods well known to a person skilled in the art. Such a random cutting prevents binding of the probe to very large target molecules, which would (partly) survive the exonuclease treatment.

For an optimal hybridisation reaction to the target nucleotide in the sample it has been found that the melting temperatures (Tm) of the two target specific sequences T1 and T2 need to be different. Further, it was found that asymmetric PLP design, in which a long 5' arm serves as an anchor sequence and the binding of a short 3' arm is an equilibrium process, could increase mismatch discrimination by almost one order of magnitude. Faruqi and colleagues also demonstrated the superiority of asymmetric PLP design (Faruqi, A.F., Hosono, S., Driscoll, M.D., Dean, F.B., Alsmadi, O., Bandaru, R., Kumar, G., Grimwade, B., Zong, Q., Sun, Z., Du, Y., Kingsmore, S., Knott, T. and Lasken, R.S. (2001) High-throughput genotyping of single nucleotide polymorphisms with rolling circle amplification. BMC Genomics, 2, e4.). Their assay conditions and evaluation method, however, were very different from those used in this invention. A further advantage of the asymmetric design is that while the 3' arm may ensure excellent specificity, the binding of the long 5' arm is quite stable and might tolerate potential mismatches caused by polymorphisms within the target group. For a sufficient and specific recognition of the target sequence in the sample, the PLP preferably comprises a 5' arm (T1), which preferably has a length of about 10 to about 75 nucleotides, more preferably of about 20 to about 50 nucleotides and most preferably of about 25 to about 40 nucleotides. The shorter 3' arm (T2) preferably has a length of about 10 to about 30 nucleotides, more preferably of about 10 to about 20 nucleotides. Examples of such T1 and T2 sequences are given in Table 3A.

The pivotal point of the present invention is that even a better detection is achieved when it is possible to isolate the circularized PLPs from the reaction mixture, which contains not only the ligated full length PLPs which have been linearized by cleavage at the unique cleavage site, but also the unreacted sample nucleotides, and short PLP fragments stemming from non-ligated, cleaved PLPs. Isolation of the circularised probes is accomplished by incorporation of a nucleotide bearing desthio-biotine. Isolation of the PLPs can then be achieved by contacting said PLPs with a solid support carrying the second member of said binding pair, and thus binding the PLPs. After binding the unreacted nucleotide sequences can be washed away, whereafter the bound PLPs can be eluted from the solid support. The solid support can be anything which is able to carry the second member of the binding pair, such as beads or a column. The material of the solid support can be any material which is conventionally used in biochemical procedures of this kind, such as glass, polystyrene, polyethylene, and the like.

The binding pair according to the invention is (desthio-)biotin/streptavidin. It has been found extremely suitable to provide the PLP with a nucleotide carrying a desthio-biotin moiety. This enables binding to streptavidin coated magnobeads (Hirsch JD, Eslamizar L, Filanoski BJ, Malekzadeh N, Haugland RP, Beechem JM and Haugland RP. (2002) Easily reversible desthiobiotin binding to streptavidin, avidin, and other biotin-binding proteins: uses for protein labeling, detection, and isolation. Anal Biochem. 2002 Sep 15; 308 (2):343-57) after which the unbound nucleotides can be washed off. The PLPs which are bound to the beads can be set free again by addition of biotin, which binds more strongly to the streptavidin coated magnobeads and replaces the PLP. With this system it is possible to obtain a rather pure isolate of the PLPs which have recognized and hybridised to a target in the original reaction mixture and have been ligated to form circular PLPs. Elution can be performed by addition of biotin.

The nucleotide bearing desthio-biotin moiety, is engineered between the unique reverse primer binding site and the unique cleavage site, since there it will not interfere with any subsequent amplification reaction (it will be at the 3' end of the reverse primer binding site and thus remain outside the sequence which is amplified). Further, it needs a minimal distance from the cleavage site.

We have found that the uracil-N-glycosidase cannot approach the uracil molecules if desthio-biotin is bound too close to these nucleotides. We experimentally found out that if 12 nucleotides or more are between uracil and desthiobiotin degradation can take place (figure 20). The use of poly T's is because this does not complicate the PLP design significantly, this in contrast with a random nucleotide sequence, which can influence the secondary structure. It is contemplated that the exact sequence of this spacer is not critical as long as it does not interfere with the secondary structure and as long as it does not interfere with the amplification reaction and/or the target hybridization reactions. Thus, preferably a stretch of at least 12 identical nucleotides is used.

Contacting the sample with beads or any other support coated with streptavidin, will cause the PLPs to bind, while the rest of the sample, inclusive all unbound nucleotides, can be removed, e.g. through washing with buffer solution or by physically separating the beads. Freeing the PLPs again from the streptavidin binding can fairly easy be accomplished by a competition reaction with biotin, which binds stronger to the streptavidin than desthiobiotin and thus will replace the bound PLPs at the streptavidine beads. These free PLPs can then be eluted from the solution and thus are obtained in a purified form.

It will be clear to a person skilled in the art that by isolating the PLPs in the above mentioned way, the subsequent amplification will be less prone to false positives.

Preferably, the steps of isolation and linearization of the PLPs, as described above, are combined. This means that the linearization of the probes, i.e. the cleavage at the poly-uracil site, is performed while the PLPs are attached to the streptavidin coated solid support.

After linearization, the PLPs are fit (may serve as template) for amplification. As explained above, the use of padlock probes in general and specifically in combination with the cleavage at the uracil-site ensures that only the PLPs are amplified which have recognized a target sequence, been able to hybridise to said target sequence and which have been ligated at said target site. Thus by amplifying only those PLPs a genuine representation of the target sequences that were present in the original sample can be obtained.

After amplification the PLPs can be assayed by using any sort of assay which is capable of recognising specific polynucleotides. Depending on the type of PLP the assay can vary (as described below). Preferably, especially in the case of multiplex amplification, the assay is performed on a (micro-)array. Depending on the choice of the PLP, the method can be qualitative or quantitative.

In accordance with a quantitative method of the invention, the specific sequence of the PLP (which can for instance be provided by the ZIP-code or by the target specific sequences T1 or T2) is recognised by a specific capture molecule (e.g. a sequence which is capable of hybridisation with said specific sequence), which bears a label. The strength of the generated signal is compared to a calibration curve produced from specific sequences in known amounts. As a result, the amount of target nucleotide sequence in the sample can be calculated. This technique thus uses an external standard.

For labelling, substances such as radioisotopes, fluorescent substances, chemiluminescent substances and substances with fluorophore, and the like may be used. For instance, the fluorescent substance includes Cy2, FluorX, Cy3, Cy3.5, Cy5, Cy5.5, Cy7, fluorescein isothiocyanate (FITC), Texas Red, Rhodamine, Alexa 532 and the like. Methods to attach the labels to the nucleotides are generally known in the art.

Alternatively, a quantitative method of the invention relates to an internal standard. In this method, a known amount of one or more marker target nucleotide sequences is added to the sample. These will then be recognised by a PLP which is specifically designed for this marker target nucleotide. The PLP will undergo the same treatment as the PLPs which have recognised their target nucleotides in the sample. When performing the assay, all PLPs are detected by their specific sequences and a comparison of the signals generated by the PLP which is directed to the marker target nucleotide with the signals generated by the other PLPs indicates the relative amount of target present in the sample. Since the concentration of the marker target nucleotide was known, the concentration of other target nucleotides can be calculated. To decrease the error margins, several different marker target nucleotides can be added to the sample in increasing concentrations, to generate a sort of internal calibration curve.

In another preferred embodiment of the invention, both the amplification and the detection of the PLPs is performed in a micro-array. The OpenArray™ technology (BioTrove, Woburn, MA, USA) currently allows parallel amplification and testing of more than 3000 assays on one plate (48 subarrays with each 64 so-called Through-Holes with a volume of 33 nL). The primers are pre-loaded into the holes, while the (purified) sample along with the reagents are autoloaded due to surface tension, provided by the hydrophilic surface of the array. Detection of the amplification can take place by simple detection of the presence of double stranded DNA. This can be done by using intercalating dyes, such as ethidiumbromide (EtBr) and SYBR Green. Alternatively, amplification products can be detected and quantified by using a universal probe, such as a TaqMan probe. TaqMan probes are probes with fluorescent dyes at opposite ends and can be used during PCR amplification. The probe is degraded during amplification by 5'-exonuclease activity of the Taq-polymerase used and increase of fluorescence can be measured real-time during amplification and in that way quantification of target is possible (see Livak KJ, Flood SJA, Marmaro J, Giusti W and Deetz K (1995). Oligonucleotides with fluorescent dyes at opposite ends provide a quenched probe system useful for detecting PCR product and nucleic acid hybridization. PCR Methods and Applications 4: 357-362).

In a preferred embodiment the TaqMan probes for detecting amplification products comprise locked nucleic acids (LNA) nucleotides. Locked nucleic acids **(LNAs)** are synthetic nucleic acid analogs that bind to complementary target molecules (DNA, RNA or **LNA)** with very high affinity. At the same time when probes are compared with and without LNA having the same Tm, binding affinity is decreased substantially for the LNA type when the hybrids thus formed contain even a single mismatched base pair. For this reason LNA existing TaqMan probes show an increased specificity (see Koshkin,A.A., Nielsen,P., Meldgaard,M., Rajwanshi,V.K., Singh,S.K. and Wengel,J. (1998) LNA (locked nucleic acid): an RNA mimic forming exceedingly stable LNA:LNA duplexes. J. Am. Chem. Soc., 120, 13252-13253).

In another preferred embodiment of the invention, the detection of the ZIP-code can be performed also directly through hybridising the said padlock nucleotide to an array or to gold beads bearing ZIP codes complementary to the nucleotide sequence in the padlock.

In the ligation detection reaction generic pre-amplified target DNA is used as a source for ligation of standard PLPs. The ligated padlock probes are hybridized on the array and labelled with e.g. a streptavidin-coupled fluorescent probe Alexa (532) directed against the desthiobiotin moiety of the padlock. This method comprises an exonuclease step after the ligation and a NaOH denaturation step before capturing of the padlock nucleotide probes. The elution can be performed with a 80 °C step in H₂O or with biotin. If the unique cleavable sequence is a poly-uracil sequence, cleavage can preferably be affected by treatment with uracil-N-glycosidase and endonuclease IV (see Fig 21 and Fig 22).

The presence or absence of ligated padlock probes can also be visualized with BCA (bio-bar-code amplification, Jwa-Min Nam *et al., supra*). BCA is a PCR-less target amplification method that relies on novel two-component oligonucleotide-modified gold nanoparticles (NPs) and single-component oligonucleotide-modified magnetic microparticles (MMPs) and subsequent detection of amplified target DNA in the form of bar-code DNA using a chip-based detection method (see Fig 23).

In this BCA detection reaction, target DNA is used as a source for ligation of standard PLPs. This method comprises an exonuclease step after the ligation and a NaOH denaturation step before capturing of the padlock nucleotide probes. The second member of a binding pair will bind the first member of a binding pair which is available on the PLP. If said first member is desthiobiotin, said second member is streptavidin. If the unique cleavable sequence is a poly-uracil sequence, cleavage can preferably be affected by treatment with uracil-N-glycosidase and endonuclease IV. The linear PLP stays bound to the streptavidin, the goldbeads bearing both ZIP codes complementary to the nucleotide sequence in the padlock and fluorescent barcode nucleotides, will hybridize (Fig. 24-25) Recently with the use of Nanoparticle-Based Bio-Bar Code technology proof- of- principle has been shown that both DNA and protein can be detected with PCR sensitivity without enzymatic amplification reaction( Christine D. Keating. Nanoscience enables ultrasensitive detection of Alzheimer's biomarker. PNAS, Feb 15, 2005, 2263-2264).

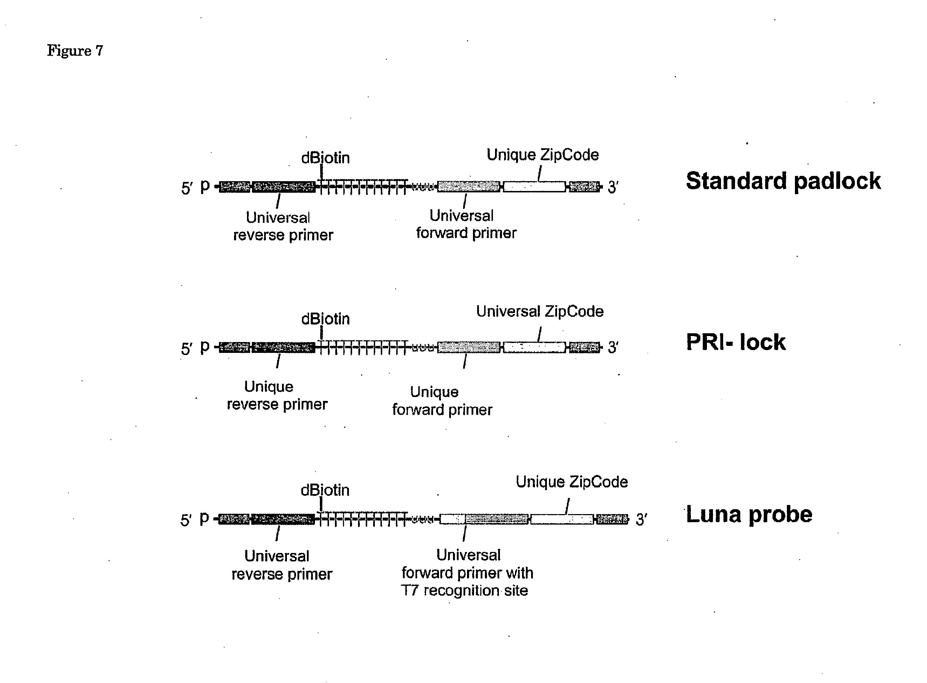

Typical preferred PLPs of the invention are the standard Padlock probe, the PRI-lock probe and the LUNA-probe as depicted in Fig. 7. The probes can be constructed using normal genetic engineering techniques, such as disclosed in handbooks like Sambrook, J., Fritsch, E.F., and Maniatis, T., in Molecular Cloning: A Laboratory Manual. Cold Spring Harbor Laboratory Press, NY, Vol. 1,2,3 (1989). Oligonucleotides which need to be assembled for use in the present invention can be made synthetically by standard DNA or RNA chemical synthesizers or may be obtained from enzymatic digestion of wild-type, naturally occurring sequences. It is also possible that naturally occurring sequences are modified by insertion, substitution or deletion of one or more nucleotides using conventional genetic engineering techniques.

In the general description of these probes herein below and in the Examples, a poly-uracil sequence is taken as the unique cleavage site, and a desthiobiotin moiety is taken as a first member of a binding pair. It should however be understood that alternatives for these embodiments, as discussed above, are also within the scope of this invention.

Insertion of the poly-uracil sequence as described above can be done by standard techniques, as mentioned above, which techniques are well known to a person skilled in the art.
It is preferred that a commercially obtained desthiobiotin moiety is used.. These moieties are commercially available as desthiobiotin coupled to a thymidine nucleotide. This nucleotide is introduced into the padlock nucleotide probe according to standard methods.

Below the working of these probes in the present invention will be explained in more detail.

### The basic principle - the standard Padlock probe

Standard padlock probes (PLPs) according to the general structure as depicted in the top figure of fig. 7 are designed in such a way that a set of those PLPs all comprise the same universal forward and reverse primer binding sites, designated as universal forward and reverse primer binding sites. Choice of these primer binding sites is flexible, insofar that care should be taken that the primer binding site sequences differ substantially from the rest of the padlock probe, so that the amplification step, which makes use of the universal primers is not hampered by homologous sequences in the rest of the probe.

In each standard PLP a unique set of target specific sequences is inserted, which is designed to bind to a specific target sequence which is suspected to be present in the sample. The target specific sequence should be unique, meaning that it can hybridise with only one target nucleotide molecule in the sample. Of course, care should be taken that the target specific sequences T1 and T2 are directed and designed in such a way that, upon hybridisation with the target nucleotide in the sample, they are able to be ligated to each other. This in particular means that either the T1 sequence should be in the sense direction (i.e. recognising the target in the 5' to 3' direction) and the T2 sequence in the anti-sense direction, or vice-versa. Further, the PLP comprises a unique ZIP-code, which eventually will serve for the detection of the PLP. There is no functional restriction with regard to this ZIP-code, other than that each PLP of the set of PLPs should have a unique ZIP-code and that it can serve for detection in the assay. For this purpose, preferably the ZIP-codes used for a given set of PLP-probes should be of the same size and character, in order not to influence the other steps of the method, such as the amplification step. Preferably, the ZIP codes are derived chosen from the GeneFlex™ TagArray set (Affymetrix) or any other similar library.

The other elements of the PLP (uracil-site, desthio-biotin moiety) are as described above.

Once a set of these probes is produced they can be added to a sample under conditions which are optimal for alignment and hybridisation of the target specific sequences T1 and T2 to the target sequences in the sample. When the hybridisation reaction is complete the PLPs that have hybridised to a target sequence are ligated by addition of the enzyme ligase to the reaction mixture. Thereafter, preferably the non-ligated DNA is removed by exonuclase degradation. This exonuclease treatment can be performed with either a 3' to 5' exonuclease or a 5' to 3' exdonuclease or both or an exonuclase wich combines both activities. It is paramount for the present invention that these exonuclease(s) do not have any endonuclease activity.

Next, the probes are captured using e.g. streptavidin coupled magnetic beads (or another streptavidin coated solid support, such as a column or filter upon which streptavidin is immobilised) and separated from the sample. Subsequently, the probes are cleaved at the uracil-site by adding a sufficient amount of uracil-N-glycosidase and endo IV nuclease.

This in particular means that ZIP-code probe region of the unligated padlock probes is removed, while the ligated probes are linearized. The probes are then eluted from the beads by using an aqueous solution of biotin or a 80°C treatment in H₂O.

Finally, the eluted probes are amplified with PCR using the universal primers (one of which is labelled). Amplicons are then hybridised on e.g. micro-arrays on which sequences which are complementary to the ZIP sequences are spotted.

### The PRI-lock probe

The general structure of the PRI-lock probe is given in the middle section of Fig. 7, with the remark that the universal Zip-code is an optional feature, as will be explained below. Note that the difference with the above standard Padlock probe is that now the primer binding sites are unique primer binding sites, while the optional ZIP-code is universal.

All the other elements of the PRI-lock probe are similar to those of the standard Padlock-probe and can be applied as mentioned above. Also the hybridisation, ligation, linearization and elution of the PRI-lock probe is identical to those described above.

The Universal ZIP-code is designed for being able to hybridise to a universal probe, such as a TaqMan probe.

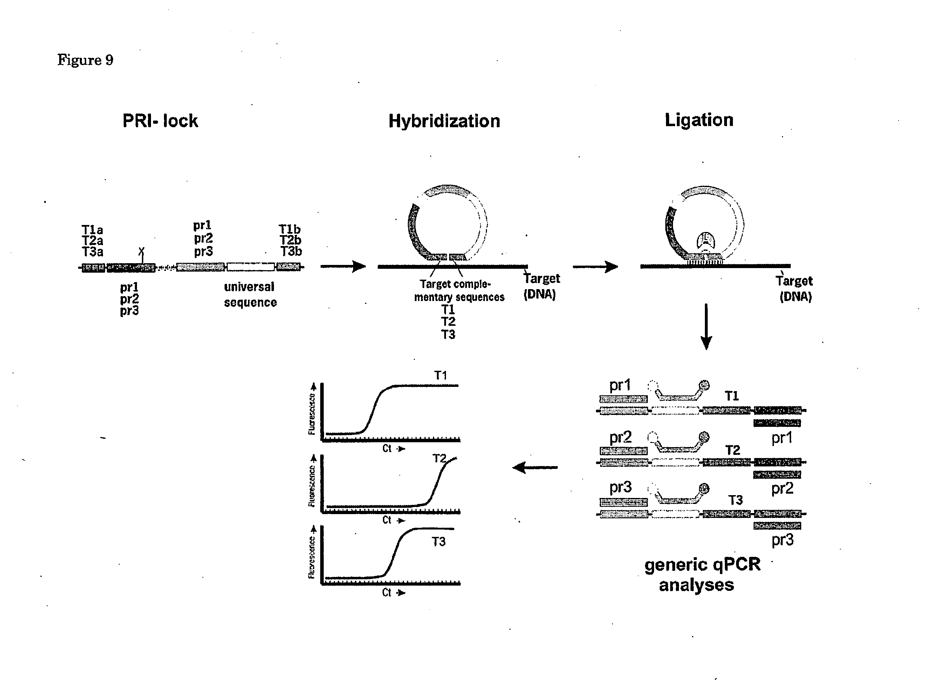

The scheme of the applied procedure is outlined in Fig. 9. Multiple PRI-lock probes are ligated on fragmented target DNA. Target recognition is achieved by specific hybridization of both arm sequences, and efficient ligation occurs only if the end nucleotides are perfectly matching to the target. Therefore, the probes confer superior specificity. After ligation, the probes are captured on streptavidin-coated magnetic beads via the desthiobiotin, and are cleaved at the deoxy-uracil nucleotides. The ligation mix and the TaqMan probe region of unligated probes are removed by several washing steps, eliminating the background due to the presence of unligated probes. The remaining probes are eluted in aqueous biotin solution or after a 80° C incubation step, the ligated probes are assayed in real-time PCR using a unique primer pair for each target.

The linear quantification range of the proposed procedure is dependent on both the ligation step and the real-time PCR. Ligation of oligonucleotides has been shown to reflect well the target quantity and was used successfully for characterization of gene expression and gene copy number in a multiplex setting.

PRI-locks combined with the OpenArray™ system (BioTrove) are useful for a flexible and easily adaptable design of high-throughput, quantitative multiplex DNA assays, since the target recognition step is separated from downstream processing. The primer binding and TaqMan probe sites were chosen from a set of artificial, well-balanced sequences that had been selected to have minimum cross-hybridization (e.g. the GeneFlex™ TagArrays set (Affymetrix))

Such a design ensures ideal amplification under universal PCR conditions and enables the use of a single, universal TaqMan probe. Therefore, the system is cost-efficient, easily modifiable and extendable to other targets, even newly emerged pathogens.

It is also possible to omit the universal ZIP-code from the PRI-lock probe. In that case, detection of a successful amplification is achieved by detecting double-stranded DNA. There are numerous ways to detect double-stranded DNA, but in the art this is mostly achieved by using so-called intercalating dyes, i.e. dyes which only adhere to the nucleotides when these are double-stranded. The most known example of such a dye is ethidiumbromide (EtBr), but use of this dye is disadvantageous because of its toxicity. A useful alternative is SYBR Green, a commercially available dye (e.g. from Invitrogen or Applied Biosystems). Addition of the dye to the PCR mixture gives an easy read-out if double-stranded DNA is present in the mixture, which is indicative of a successful amplification, i.e. presence of the target.

### The LUNA probe

The LUNA probe is also a variant of the above described standard Padlock probe, the difference being that the universal forward primer binding site comprises a T7 polymerase recognition site. When the hybridisation reaction is complete the PLPs that have hybridised to a target sequence are ligated by addition of the enzyme ligase to the reaction mixture. Thereafter, preferably the non-ligated DNA is removed by exonuclase degradation. This exonuclease treatment can be performed with either a 3' to 5' exonuclease or a 5' to 3' exdonuclease or both or an exonuclase wich combines both activities. It is paramount for the present invention that these exonuclease(s) do not have any endonuclease activity. Next, the probes are captured using e.g. streptavidin coupled magnetic beads (or another streptavidin coated solid support, such as a column or filter upon which streptavidin is immobilised) and separated from the sample. Subsequently, the probes are cleaved at the uracil-site by adding a sufficient amount of uracil-N-glycosidase and endo IV nuclease. By addition of the reverse primer and the presence of AMV reverse transcriptase the T7 site becomes accessible for the RNA polymerase which is common for all the LUNA probes and is used as starting point for a generic NASBA amplification at a fixed temperature (Compton, J, 1991, Nature 350: 91-92.; Kievits, T, van Gemen, B, Van Strijp, D, Schukkink, R, Dirks, M, Adriaanse, H, Malek, L, Sooknanan, R and Lens, P. 1991, J. Virol. Methods 35: 273-286; Leone, G, van Schijndel, H, van Gemen, B, Kramer, FR, and Schoen, CD. 1998, Nucleic Acids Research 26: 2150-2155.). A NASBA reaction is based on the concurrent activity of AMV reverse transcriptase (RT), RNase H and T7 RNA polymerase, together with two primers to produce amplification (3). This process occurs at one temperature (41°C)

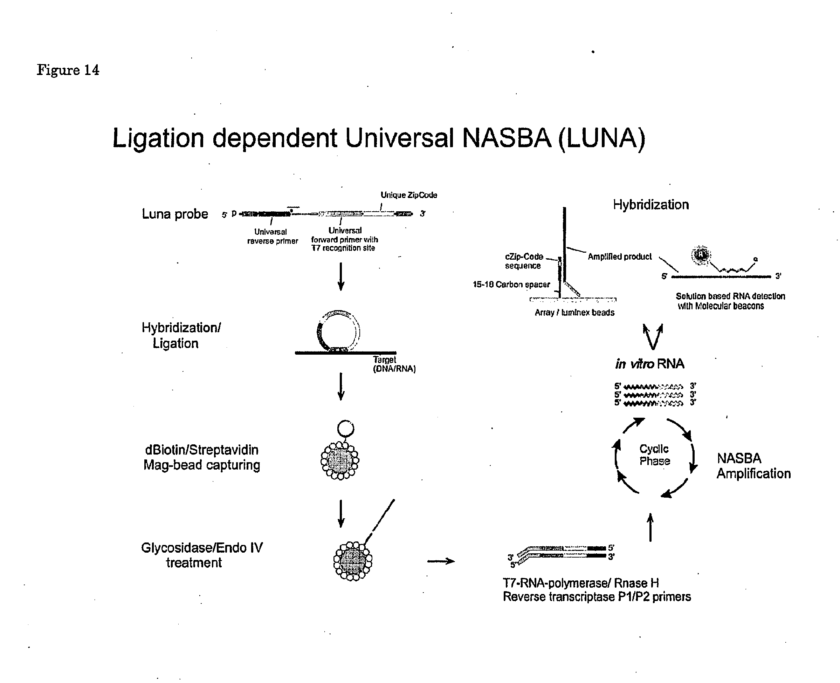

The generated ssRNA's are individually detected via the designed identifier sequences (ZIP-Code), which allows unique target dependent identification. Figure 14 depicts a generalised assay method with these LUNA probes.

Two different approaches (Fig. 19) for detection can be followed to analyse the amplification products: i) on array or ii) in solution.

### i) on array.

With the array based multiplexing approach the amplified products are collected on different matrices (array or Luminex beads) (Gordon RF, McDade RL, 1997, Multiplexed quantification of human IgG, IgA, and IgM with the Flowmetrix system. Clinical Chemistry, 43: 1799-1801) where complementary ZIP (cZIP)-Code oligonucleotides with free 3' ends have been immobilized (Fig. 14.). With arrays probe addressable sites are used for target identification. Luminex beads are color coded beads; the association of the amplified product with a characteristic Luminex bead is used as a tag for target identification. With the Luminex approach we have an array in solution. The introduction of cZIP-Code sequences (arbitrarily non-target sequence of approximately 20-25 nucleotides) makes detection of amplified products on both matrices independent from target sequences. As the array or Luminex beads containing cZIP-Codes are independent, this makes the described assay system adaptable for different fields of application.

### ii) in solution

With the solution based multiplexing approach amplification of reacted padlock probes is performed by a universal NASBA at a fixed temperature. The ZIP-Code of every different padlock probe is used as a recognition site for a molecular beacon in NASBA (Leone, G. et al, *supra*). Molecular beacons are single-stranded oligonucleotides having a stem-loop structure. The loop portion contains the sequence complementary to the target nucleic acid, whereas the stem is unrelated to the target and has a double-stranded structure. One arm of the stem is labeled with a fluorescent dye, and the other arm is labeled with a non-fluorescent quencher. In this state the probe does not produce fluorescence because the energy is transferred to the quencher and released as heat When the molecular beacon hybridizes to its target it undergoes a conformational change that separates the fluorophore and the quencher, and the bound probe fluoresces brightly (Tyagi, S. and Kramer, F.R. (1996) Nature Biotechnol., 14, 303-308).

During this amplification fluorescence based identification is performed. The combination of PLPs with NASBA is new for multiplex detection of different RNA/DNA target sequences and has been named LUNA: Ligase dependent Universal NASBA (Fig. 14). Especially the incorporation of the T7 recognition site into the PLP and the use of a universal NASBA generating ssRNA is a new feature in the development of an efficient multiplex detection method, which allows lower target detection levels. Combining this technique with microarray technology makes it a universal tool for multiplex analysis for different targets.

### EXAMPLES

### Example 1: Multiplex Diagnosis of Plant Pathogenic Micro-organisms

### Nucleic acids used in the study

The pathogenic organisms were derived from the culture collection of the applicant. (Table 1) Genomic DNAs were extracted as previously described (Bonants, P., Hagenaar-de Weerdt, M., van Gent-Pelzer, M., Lacourt, I., Cooke D. and Duncan, J. (1997) Detection and identification of Phytophthora fragariae Hickman by the polymerase chain reaction. Eur. J of Plant Pathol., 103, 345-355.).

### Padlock probe design

Relevant nucleic acid sequences derived from Genbank and from independent sequencing studies were aligned by using ClustalW, implemented in BioEdit (http://www.mbio.ncsu.edu/BioEdit/bioedit.html). Diagnostic sequences were identified for each target group. Potential PLP target complementary regions were selected in a way that the discriminatory nucleotides would bind to the 3'arm region, and to match certain stability criteria (see Results). Melting temperatures (Tₘ) to characterize binding strengths of arm sequences were calculated using the nearest neighbour method, as implemented in Hyther™ (http://ozone2.chem.wayne.edu/). The prediction parameters were set to match ligation conditions ([Na⁺] = 0.025 M; [Mg²⁺] = 0.01 M; T = 65°C and [oligo] = 2.5* 10⁻¹¹ M). Specificity was ensured by positioning a strongly destabilizing mismatch at the PLP 3'end with closely related, non-target sequences. PLPs with target oligonucleotides as listed in Tables 2A and 3A, were synthesised by Eurogentec S.A. (Seraing, Belgium). The PLP arm sequences were combined with the universal primer binding sites (P1: 5' CTCGACCGTTAGCAGCATGA 3'; P2: 5' CCGAGATGTACCGCTATCGT 3') and a ZipCode sequence. The unique identifier was chosen from GeneFlex™ TagArray set (Affymetrix) in a way to minimize PLP secondary structures. Secondary structure predictions were performed by using MFold (http://www.bioinfo.rpi.edu/applications/mfold/). When necessary, PLP arm sequences were also adjusted to avoid strong secondary structures that might interfere with efficient ligation.

### Ligation and exonuclease treatment

Genomic DNA was fragmented by digestion using EcoRI, HindIII and BamHI (New England Biolabs) for 30 min, and used as template in the indicated amount. Cycled ligation was performed in 10 µL reaction mixture containing 20 mM Tris-HCl pH 9.0, 25 mM KCH₃COO, 10 mM Mg(CH₃COO)₂, 10 mM DTT, 1 mM NAD, 0.1% Triton X-100, 20 ng sonicated salm sperm DNA, 2.4 U Taq ligase (New England Biolabs) and 25 pM PLP. For multiplex detection the concentration of the individual PLPs were adjusted to achieve comparable performance, and ranged from 25 to 200 pM. Reactions mixtures were made up on ice, and transferred rapidly to a thermal cycler. After 5 min at 95°C, 20 cycles of 30 sec at 95°C and 5 min at 65°C were performed, followed by 15 min inactivation at 95°C. After ligation, 10 µL of exonuclease mix (10 mM Tris-HCl pH 9.0, 4.4 mM MgCl₂, 0.1 mg/ml BSA, 0.5 U Exonuclease I (USB) and 0.5 U Exonuclease III (USB) was added to each reaction, and the samples were incubated at 37°C for 2 h, followed by inactivation at 95°C for 2.5 h.

### Real-time PCR

Amplification of ligated PLPs was followed in real-time using an ABI Prism 7700 Sequence Detector System (Applied Biosystems) and the qPCR kit (Eurogentec). Reaction mixtures of 25 µL contained 2.5 µL 10X real-time buffer, 3 mM MgCl₂, 200 nM of each dNTP including dTTP/dUTP, 100 nM P-Frag TaqMan probe (5' FAM-CCCGGTCAACTTCAAGCTCCTAAGCC-TAMRA 3'), 300 nM of primers P1-f20 (5' CCGAGATGTACCGCTATCGT 3') and P2-r20 (5' TCATGCTGCTAACGGTCGAG 3'), 0.6 U Hot Gold Start polymerase, 0.6 U UNG and 3 µL ligation-exo mix as template. The reaction mix was initially incubated at 50°C for 2 min, followed by 10 min denaturation at 95°C, and 40 cycles of 15 sec at 95°C and 1 min at 60°C. Fluorescence was recorded in the second step of each cycle.

### LATE-PCR

For microarray hybridisation, circularised PLP probes were amplified in LATE-PCR (linear-after-the-exponential PCR) (Sanchez, J.A., Pierce, K.E., Rice, J.E. and Wangh, L.J. (2004) Linear-after-the-exponential (LATE)-PCR: an advanced method of asymmetric PCR and its uses in quantitative real-time analysis. Proc. Natl. Acad. Sci. U S. A., 101, 1933-1938.) to produce a large amount of ssDNA amplicons. Lengths of the primers were adjusted so that they would have similar melting temperatures despite the 10-fold concentration difference. PLPs were amplified in 25 µL reaction mixtures containing 1X Pfu buffer (Stratagene), 200 nM of each dNTP, 500 nM of Cy3- or Cy5-labeled P1-f19 primer (5' CGAGATGTACCGCTATCGT 3'), 50 nM P2-r20 primer, 0.375 U Pfu (Stratagene) and 3 µL ligation-exo mix as template. The temperature profile of the reaction was: 5 min at 95°C, 40 cycles of 2 sec at 51°C, 5 sec at 72°C and 15 sec at 95°C, after which the reaction was immediately cooled to 10°C. PLP amplicons were analysed by agarose gel electrophoresis before applying them on array.

### Microarray preparation

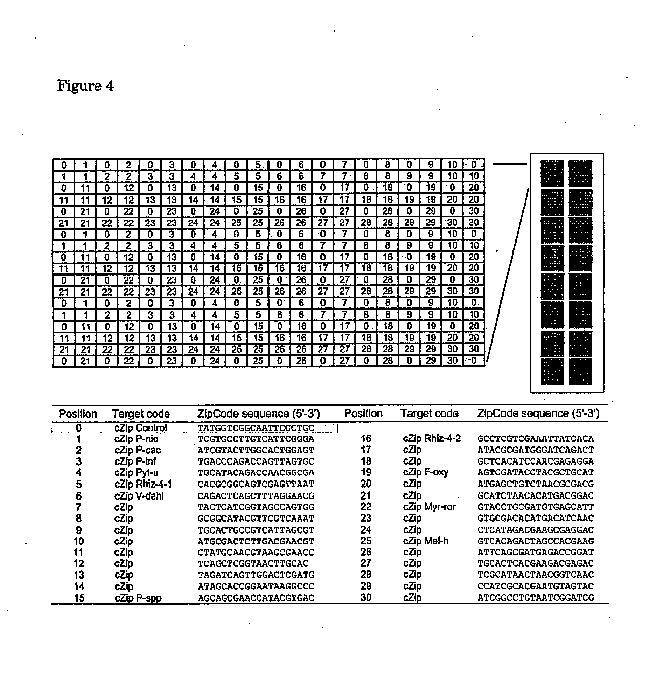

Complementary ZipCode (cZipCode) oligonucleotides (Fig. 4) carrying a C12 linker and a 5' NH₂ group were synthesised and spotted on Nexterion MPX-E16 epoxy-coated slides by Isogen B.V. (Utrecht, The Netherlands) according to manufacturer's instructions (Schott Nexterion). Briefly, 50 nL of 1.5 mM cZipCode solution was spotted using an OmniGrid100 contact-dispensing system (Genomic Solutions) equipped with SMP4 pins (Telechem) at 50% relative humidity. After 1 hour incubation at 75% humidity, the uncoupled probes were removed by washing in 300 mM bicine, pH 8.0, 300 mM NaCl, and 0.1% SDS for 30 min at 65°C, followed by rinsing with deionised water and drying by spinning at 250 g for 2 min. The arrays were stored in dark, in a desiccator at room temperature until use.

### Microarray hybridisation

Prior to hybridisation, the arrays were washed and the functional groups were blocked according to manufacturer's instructions. The hybridisation mixes were made up of 5 µL Cy3-labeled sample and 5 µL Cy5-labeled background control sample in 3 M TMAC, 0.1 % sarkosyl, 50 mM Tris-HCl pH 8.0, 4 mM Na₂EDTA. Cy5-labeled hybridisation control was added to 20 pM final concentration in 50 µl final volume. For each slide, one of the hybridisation samples contained Cy5- and Cy3-labeled amplicons corresponding to the same, positive ligation reaction, which served to correct for dye bias (dye correction sample). The mixes were heated for 10 min at 99°C and cooled down rapidly on ice. Sixteen-well silicon superstructures (Schott Nexterion) were attached to the arrays to create separate chambers for the subarrays. After adding 40 µl of the samples to each well, the chambers were sealed, and the arrays were hybridised at 55°C o/n in high humidity. Afterwards, the isolators were removed, and the slides were washed once at 55°C for 5 min in prewarmed 1XSSC/0.2% SDS, and twice for an additional 1 min at RT in 0.1XSSC/0.2% SDS and in 0.1XSSC, respectively. Finally, the slides were dried by spinning at 250 g for 2 min.

### Analysis of microarray data

Microarrays were analysed using a confocal ScanArray^{®} 4000 laser scanning system (Packard GSI Lumonics) containing a GreNe 543 nm laser for Cy3 and a HeNe 633 nm laser for Cy5 fluorescence measurement. Laser power was fixed at 70% for both lasers, while PMT (photomultiplier tube power) ranged from 45 to 65%, depending on signal intensity. Fluorescent intensities were quantified by using QuantArray^{®} (Packard GSI Lumonics), and the parameters *'mean signal - mean local background'* (*mean Cy3-B or mean Cy5-B*) and the *'mean local background' (B)* were used in further calculations. *Dye correction factor* was calculated separately for each slide and scanner setting, based on the subarray to which the same but differently coloured samples were hybridised (averaged *(mean Cy3-B)*/*(mean Cy5-B)* based on positive spots). *Assay background* for the other subarrays was calculated per spot as *'mean Cy5-B multiplied by the dye correction factor'. Absolute signal intensity* was defined as *mean Cy3-B minus assay background'* and was transformed to log scale *(signal = log₂(absolute signal)).* If *'mean Cy3-B'* was lower than *'assay background'* signal was evaluated as zero. To evaluate the significance of the signal, we compared it to the corresponding assay background, and calculated *log₂(absolute signal*/*assay background),* which was called *reliability factor.* The probes were spotted in 3 times triplicates (9 parallels). After excluding the outliers, signals and reliability factors were averaged for the probes, and standard deviations (SD) were calculated. A signal for a probe was called positive if the reliability factor was higher than 1 (i.e. signal was minimum twice the assay background) and the mean Cy3 signal was higher than twice the mean local Cy3 background (cut-off value).

### Results

### Evaluation of ligation specificity and PLP design strategies

For diagnostic applications, the high discriminatory power of the ligation is of prime importance, since very similar, non-target DNA molecules can be present potentially in much higher concentration than the target DNA. Therefore, we aimed to optimise the reaction conditions and PLP design for maximum discrimination of single mismatches, which subsequently could be extrapolated to diagnostic assay design.

To characterize the discriminatory power of ligation under assay conditions, we quantified the circularised PLPs by real-time TaqMan PCR (Fig. 1). The quantification range of the real-time PCR was linear over a minimum of five orders of magnitude and the amplification efficiency (E) was found to be 0.81 (Fig. 2, insert).

The experimental system to optimise the ligation conditions consisted of PLP P-frag, which targeted the ITS region of *Phytophthora fragariae,* and of the corresponding synthetic, target and non-target oligonucleotides (Table 2A). First, we tested various reaction conditions, and found that cycled ligation consisting of 20 cycles of 5 minutes at 65°C provided good discrimination, sufficient yield of ligation product and freedom from potential secondary structures (data not shown). The reaction mixture also contained 20 ng sonicated salmon sperm DNA, which served to provide a large excess of non-target DNA. All the subsequent experiments were performed under these conditions. Using oligonucleotides D0 - D6 as targets, we tested how the discriminatory power of PLP P-frag depended on the type and the position of the mismatch (Table 2B). The discrimination factor was defined as the fold-difference in the yield of ligation product with target and mismatched oligonucleotides, as determined by real-time PCR. In agreement with previous results (10), mismatches positioned at the 3' end of PLP were strongly discriminating, while those at the 5' end provided much less specificity. The type of the mismatch was also found to be important, although to lesser extent. In general, it appeared that the nearest neighbour parameters could be indicative of the destabilizing effects of different mismatches. Mismatched nucleotide pairs including cytosines were better discriminated, while the G-T pair (at the 5' end) hardly affected the ligation efficiency.

Next, we examined whether different PLP design strategies could further improve the discriminatory power. Apart from the above-described symmetric design, we tested two asymmetric design principles. Since we used the same PLP, the effect of variable arm lengths was mimicked by changing the probe-complementary sequence of the target oligonucleotides (Table 2A). First, we shortened the probe-complementary sequence to the 3'arm to increase its discriminatory power, with a corresponding lengthening of the 5' arm-complementary sequence to ensure the stable binding of the probe (oligonucleotides A1 and A1C). The second strategy involved inserting a destabilizing mismatch in the middle of the 3' arm-complementary sequence, and the binding of the probe was similarly stabilized by lengthening the 5' arm (oligonucleotides A2 and A2C). As a consequence, the melting temperatures (Tₘ) of the 5' arm sequences became higher than the reaction temperature, while those of the 3' arms were about 20 to 30°C below it (Table 2C). We believe that these Tₘ values are only indicative of the real binding conditions, since the hybridisation of the 5' arm of PLP makes the binding of the 3' arm almost a unimolecular reaction. We hypothesized that such design principles would result in an equilibrium process between a bound and an unbound 3' arm, which could increase specificity. As expected, both asymmetric design strategies significantly increased the discriminatory power of PLP P-frag against non-target oligonucleotides with 3' C-C mismatches (Table 2C and Fig. 2). Shortening the 3' arm sequence proved to be more efficient, since it did not reduce the ligation yield as much as the internal mismatch. Using this strategy we could achieve a discrimination factor of 1477 as compared to 175, provided by the symmetric design. Therefore, we selected this PLP design to generate probes for our diagnostic system to detect plant pathogenic organisms. It is interesting to note that although the internal mismatch resulted in less decrease in Tₘ than the strategy involving a short 3' arm sequence, it reduced the ligation efficiency more. We believe this phenomenon is due to the perturbation of dsDNA secondary structure, which could hinder the ligation reaction.

### Design and testing of diagnostic padlock probes

Based on the principles described above, we designed PLPs targeting ten, economically important plant pathogens (Table 3A). In each case, we selected discriminatory areas within the ITS regions of rRNA operons because of their high copy number (1), which could significantly increase the sensitivity of the assay. Further, ITS regions have been extensively used in phylogenetic studies (11), and a large number of sequences are available for plant pathogenic organisms, which may ensure reliable assay design. Sequences available in Genbank and those obtained from independent sequencing studies were aligned, and diagnostic regions for each target organism were selected. Preferably, we chose regions containing more than one discriminatory nucleotides, and very few polymorphic positions within the targeted species/subgroups. The 3' arm sequences were selected to be 14-18 nucleotide-long and had a Tₘ around 40°C (Table 3B). In general, the 3' arm sequence hybridised to the discriminatory region and contained a highly destabilizing mismatch or a gap at the 3' end when bound to the non-target sequence. The 5' arm sequences were 27-37 nucleotide-long. As an attempt at hierarchical diagnostic analysis, we also designed a genus-specific PLP to target all *Phytophthora* species and discriminate them from related *oomycetes.* After selecting the target-complementary regions, they were combined with the universal primer binding site sequences, and a unique ZipCode sequence was selected for each probe.

The developed probes were tested for sensitivity and discriminatory power using synthetic oligonucleotides representing target nucleic acids and the most similar, non-target sequences. Our rationale to test PLPs with oligonucleotides was that it was easy to implement and provided reliable data to compare PLP properties. Further, identification of certain subtypes often requires extensive characterization, while other isolates, mostly those of the closely related non-target organism, might be exotic and difficult to obtain. Therefore, we propose that an initial testing of PLPs with target and non-target oligonucleotides could be adapted as a standard approach.

Since the final analysis was to be performed on array, we chose the LATE-PCR protocol (Sanchez, J.A. et al. *supra*) to achieve efficient amplification and produce large amount of ssDNA in one step, which is ideal for microarray hybridisation. In all the subsequent experiments this method was used to amplify ligated PLPs.

Fixed amounts of PLPs were ligated on their respective target and the related, but non-target oligonucleotides, present in a wide concentration range (Fig. 3A). Sensitivity of PLP detection was defined as the lowest amount of target oligonucleotide that resulted in a positive signal as assessed by gel electrophoresis. The magnitude difference between the lowest amount of the target and that of the non-target oligonucleotide that gave positive signal was called 'discriminatory range'. These experimental characteristics along with the design parameters for each PLP are shown in Table 3B.

In order to extrapolate these values to the expected specificity in real-world assays, we also tested PLP sensitivity and discriminatory range with a dilutions series of genomic DNA. Since the isolate of *Pythium splendens* (Genbank accession no. AF310336) that is the most similar non-target organism for PLP Phyt-spp was not available, we performed this experiment with PLP P-cac, which discriminates *P. cactorum* and *P. nicotianae* species based on two nucleotides. Both PLP P-cac and PLP P-nic could successfully detect their targets using 5 pg of genomic DNA, which corresponds to ∼100 genome equivalents (Kamoun, S. (2003) Molecular genetics of pathogenic oomycetes. Eukaryot. Cell, 2, 191-199) (Fig. 3B). This experiment proved that the probes have similar and sufficient sensitivities, and the genomic DNAs were of good quality. However, ligation of PLP P-cac even on very high amounts of *P. nicotianae* genomic DNA (250 ng) did not give rise to any discernible PLP amplicons, indicating strict specificity (Fig. 3C). Since the discriminatory range of PLP P-cac was among the lowest of those of the designed PLP set, we concluded that all the probes must be specific to their cognate genomic DNA target.

### Validation - PLP-based multiplex detection of plant pathogenic organisms

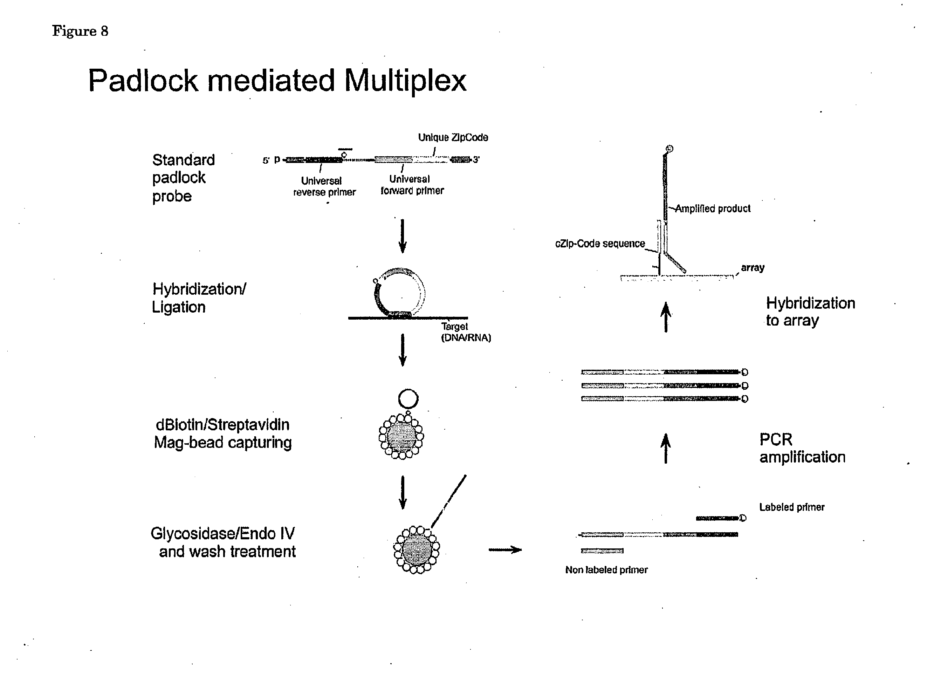

A mix of the developed 11 PLPs was ligated on various genomic DNAs, treated with exonucleases, and subjected to LATE-PCR using Cy3-labeled forward primer. The labelled PLP amplicons were analysed on multi-chamber, low-density universal microarrays, which enabled the simultaneous assay of 16 samples on a single slide (Fig. 4). The tag array used in our experiments contained 30 probes in 9 replicates, together with 90 hybridisation control probes distributed over the deposition area. This layout allows for the future extension of the PLP set to target other pathogens and enables high-throughput screening (see Fig. 8).

Because of the great sensitivity of microarray detection, we found that significant fluorescent signal could be detected even when target DNA was absent from the ligation reaction. Since our results indicated that the ligation reaction is highly specific, we concluded that the observed signal must have been derived from amplification of unligated PLPs that had not been completely removed by exonuclease treatment ('background amplification'). This 'assay background signal' was comparable to that measured in the absence of ligase, suggesting a ligation-independent mechanism (data not shown). To correct for the ligation-independent signal and to define the detection threshold of the assay, we incorporated a background control sample, which contained no target DNA in the ligation and was subjected to the same treatment. It was labelled with Cy5 and hybridised to each array together with the Cy3-labeled PLP amplicons. The assay background signal, measured in the Cy5 channel and corrected for dye bias, was deducted from the Cy3 signal for each spot. Further, we calculated a reliability factor characterizing the ratio of signal and assay background (log₂ (absolute signal/assay background)).

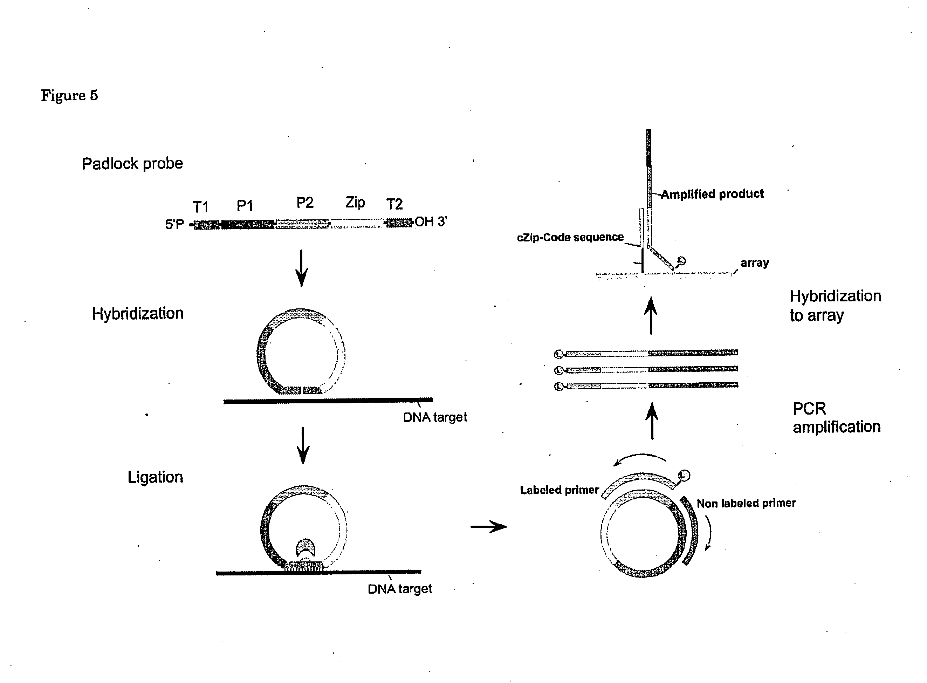

Using the developed PLP set, we tested genomic DNAs from a panel of well-characterized isolates of plant pathogenic organisms (Tables 1 and 4; Fig. 5 a-g). In each case, 1 ng genomic DNA could be specifically and reliably detected without any cross-reaction. All the *Phytophthora* species were correctly recognized by PLP Phyt-spp, including *P. cactorum,* which contained two adjacent mismatches with the 5' arm sequence of the probe (Table 3A). This polymorphism was apparently well tolerated, resulting in a positive signal. For four probes (PLPs P-cac, P-nic, P-inf and V-dahl) analysis was also performed with DNA of a very closely related organism (Table 3B), but no cross-reaction was observed, indicating excellent specificity. When there was no cognate target DNA present to any of the PLPs, we observed a certain level of Cy3 signal for some of the probes. They were, however, well below the threshold, correctly identifying the samples as negative. In the presence of ligation target, PLPs were circularised and could serve as template in PCR. Consequently, amplification of the ligated PLPs proceeded efficiently, suppressing the ligation-independent amplification, and the corresponding signal for the non-cognate probes was reduced. Thus, using the above-described correction method, zero signal was scored for most probes when there was ligation target present, due to subtraction of assay background signal.

Next, we evaluated the ability of the developed diagnostic system to detect several pathogens in parallel. Mixtures of equal amounts of genomic DNAs representing three targeted organisms were tested (Fig. 5 h-j). In two out of three cases, the pathogens were correctly and unambiguously identified by all four cognate probes, resulting in detection at the genus and species/subgroup level. The components of the third mixture, *P. cactorum, R. solani AG 4-1 and V. dahliae,* were also correctly identified by using the species/subgroup-specific probes. The PLP Phyt-spp signal, however, was below the threshold, most probably due to the two adjacent mismatches with *P. cactorum* DNA.

To explore the sensitivity of the system in a multiplexed setting, we tested the detection threshold for *F. oxysporum* and *M. roridum* in the presence of a large excess of the other target DNA (Fig. 5 k-l). As little as 0.5 pg of *F. oxysporum* DNA could be detected in the presence of 500 pg *M. roridum* DNA, corresponding to a dynamic range of 1000. In a reverse situation, the detection threshold was 5 pg for *M. roridum* in the presence of 500 pg *Fusarium* DNA, indicating that a reciprocal dynamic range of 100 is achievable using this system.

### EXAMPLE 2

### Pilot-scale study to demonstrate proof-of-principle PRI-lock probes

### Design

Three PRI-lock probes were designed to target economically important plant pathogens so as to create a pilot-scale, multiplex detection system to test the proposed principle (Fig. 7). A universal TaqMan probe, containing LNA (locked nucleic acids) was designed to monitor the amplification. The specificity of the assay will be demonstrated by testing DNAs of the most similar, non-target organisms for each probe (Table 5). Since target discrimination is achieved based on only a single or a few nucleotides, this pilot system also shows the potential of PRI-Locks for extremely specific, quantitative analysis on a universal platform.

### Specificity of PRI-Locks in singleplex reactions

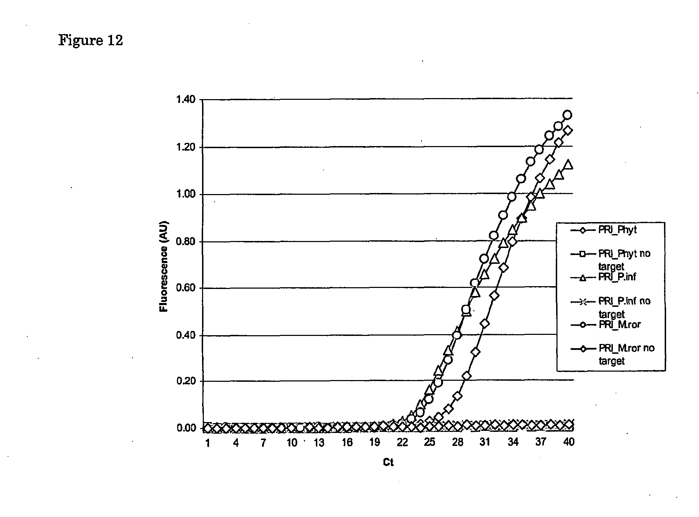

First, we tested the specific ligation of the designed PRI-lock probes on their cognate targets, and the specific amplification by using the unique primer pairs in combination with the universal TaqMan probe. Each PRI-lock probe was ligated on 500 pg target genomic DNA and specific amplifications were observed with Ct values of 22-25, depending on the PRI-lock probe used (Fig. 12). No signal was observed in lack of target DNA.

### Performance of PRI lock-based detection in a multiplex setting

To evaluate the performance of PRI-lock based detection, we carried out multiplex reactions with single or multiple target pathogens (Table 6). The Ct values observed in real-time PCR were compared to those obtained in singleplex reactions.

All the pathogens present were specifically detected without exception. The Ct values apparently were not affected by the presence of multiple probes. Further, multiple target DNAs could also be detected without substantial change in the observed Ct values, demonstrating the lack of inhibition due to possible competition in the assay. Practically, it means that the dynamic range of detection (the highest ratio of targets that is still detectable in a multiplex reaction) is not an issue using that system. We also found that the primers were perfectly specific to their respective ligated PRI-lock probe template, as expected.

*Preliminary experiments to characterize the linear range of quantification.*

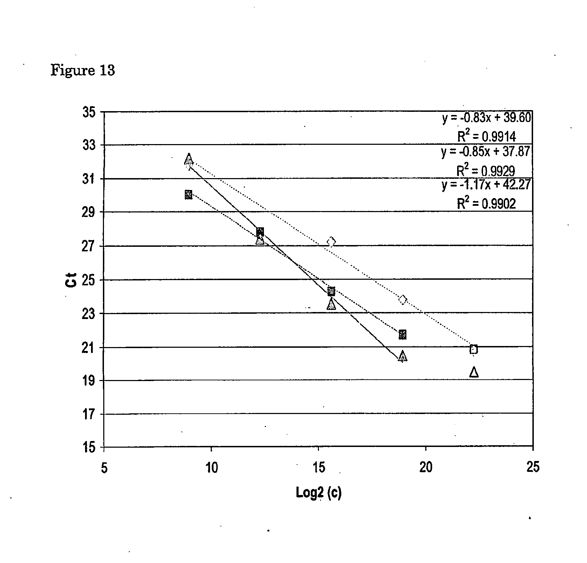

The linear range of quantification was analyzed for all the three PRI-lock probes using dilution series of target DNA. The resulting calibration curves can be used for quantification of target in subsequent experiments. Currently, we observed that for two out of the three PRI-lock probes, the linear range of quantification is only 4 magnitudes, because at higher target concentrations the ligation yield does not increase any more in a linear fashion with the increasing target concentration (Fig. 13). In the planned future application, a substantial increase in the applied PRI-lock probe concentration (100x) is expected to significantly increase the quantification range.

### EXAMPLE 3

### Pilot-scale study to demonstrate proof-of-principle LUNA probes

### Background

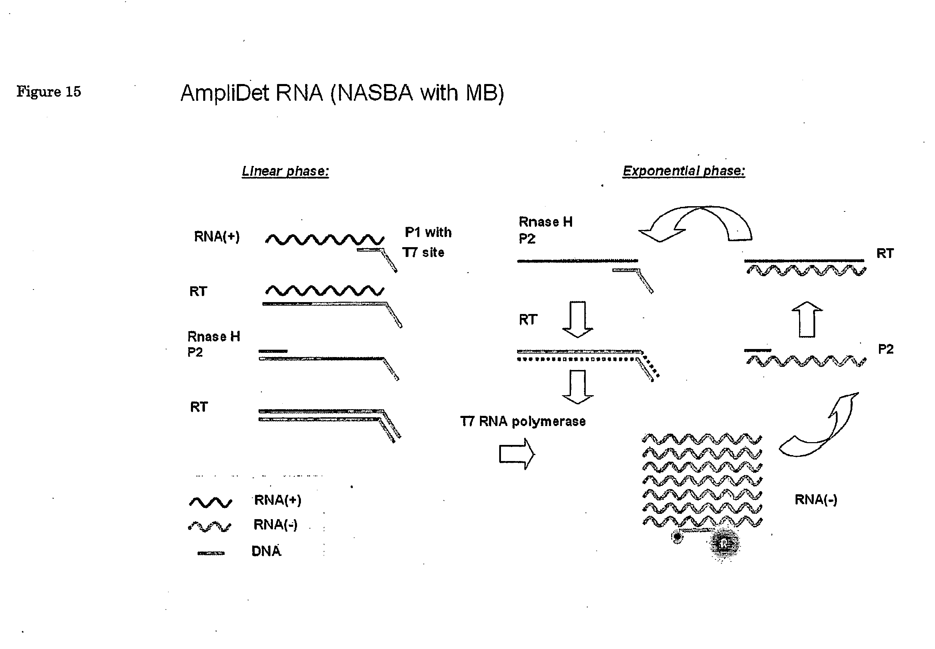

After the ligation of the LUNA probe on target DNA, any remaining non-circularized (unligated) padlock probes can be removed by exonuclease treatment followed by capturing of the probes through by binding of the desthiobiotin moiety with streptavidin magnobeads. After a washing step the remaining circularized probes are digested with Uracil-N-glycosidase/ endo IV nuclease at the position of the incorporated uracil nucleotides between the 5' T7 RNA polymerase recognition site and the 3' end of the universal reverse primer binding site. The release of the complementary T7 site, common for all padlock-probes, acts as starting point for a generic NASBA amplification at a fixed temperature (see Fig. 14-15)

### Design

According to procedures as described above (see Example 1), LUNA probes as shown at the bottom of Fig. 7 were designed.

As depicted in Fig. 14, LUNA probe hybridization, ligation, exonuclease and glycosidase treatment were performed as described previously.
Then circularized and linearized LUNA probes are amplified by standard NASBA utilising the T7 primer binding site included in de LUNA probe. Amplified ss products can be hybridized to an array or Luminex beads on which cZipCode sequences are spotted/bound. Luminex beads can be analyzed with flow cytometry.
Amplification of ligated LUNA probes in this example is measured using Molecular Beacons.

### Specificity

Based on sequences of *Phytophthora cactorum* and *Verticillium dahliae* two point mutation specific LUNA probes have been designed. A mutation on the 3'end of the probe is more discriminatory than when a mutation is placed at the 5' end. Specificity of the probe is largely increased with an asymmetrical design and a high ligation temperature. In an optimal design the 3' arm of the LUNA probe has a melting temperature (Tm) of 37-40 °C, the 5'-arm has a Tm of 65 - 70 °C. The specificity of the LUNA ligation step has been validated with closely related (non)pathogenic species and appeared to point mutation specific.

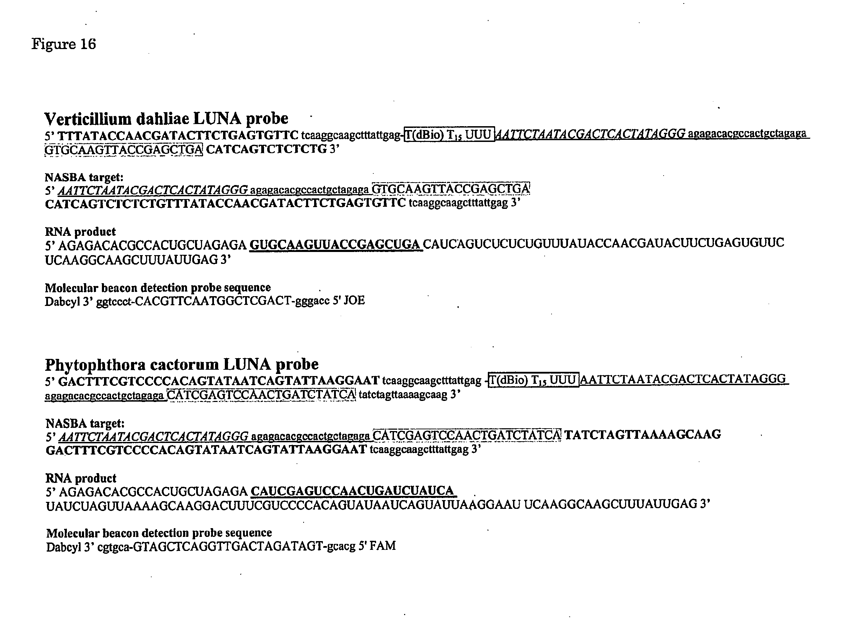

The secondary structure and in particular the localization of the T7 polymerase recognition site of the LUNA probe is essential for an efficient initiation of the NASBA amplification. Linearization of the LUNA probe by Uracil-N-glycosidase / endo IV nuclease followed by selective capturing of the probes with Streptavidine coated magnobeads appeared to be essential for an efficient NASBA amplification reaction. The target specific Zip-Codes of the LUNA probes have been used as hybridization sites for the used Molecular Beacons. With those identification tags quantification of the isothermal NASBA could be followed. As identifiers of the two LUNA probes against *P. cactorum* and *V. dahliae* a FAM- and JOE-labeled MB respectively have been designed (Fig. 16).

### Performance

The multiplexibility and dynamic detection range are important parameters of this LUNA detection system. To validate those parameters genomic DNA of the plant pathogenic *Phytophthora cactorum and Verticillium dahliae* have been extracted and tested in different concentrations and ratio's and compared with traditional PCR (Fig. 17, 18).

Multiplexing of both targets appeared to be possible; the detection limit for both targets appeared to be in the pg range (Fig. 17). The dynamic detection range for those pathogens was at least 100 (Fig. 18). For target identification the next step will be hybridization of the ssRNA LUNA amplicons to arrays spotted with cZipCode oligos or to different Luminex beads coupled with different cZipCode oligos. Detection can then be performed by array scanning, flow-cytometry or Molecular Beacon detection (Fig. 19).

### TABLES

**Table 1. Isolates of plant pathogenic species and subgroups used in Example 1.**

| Phylum | Order | Species | Isolate |
|---|---|---|---|
| Oomycota | Peronosporales | *Phytophthora nicotianae* | PRI 28.8 |
| | | *Phytophthora cactorum* | PRI 18.1 |
| | | *Phytophthora infestans* | VK98014 |
| | | *Phytophthora sojae* | F. Govers 6497 |
| | | *Pythium ultimum* | N2001/5 |
| Basidiomycota | Ceratobasidiales | *Rhizoctonia solani AG 4-1* | PRI 4R91 |
| | | *Rhizoctonia solani AG 4-2* | PRI 4R22 |
| Ascomycota | Hypocreales | *Fusarium oxysporum f. sp.* | 364N2 |
| | | *radicis-lycopersici* | |
| | | *Myrothecium roridum* | PRI 15.2 |
| | Phyllacorales | *Verticillium dahliae* | 809.97 |
| | | *Verticillium alboatrum* | Vet98/resp.VD5 |
| Nematoda | Tylenchida | *Meloidogyne hapla* | HBA |

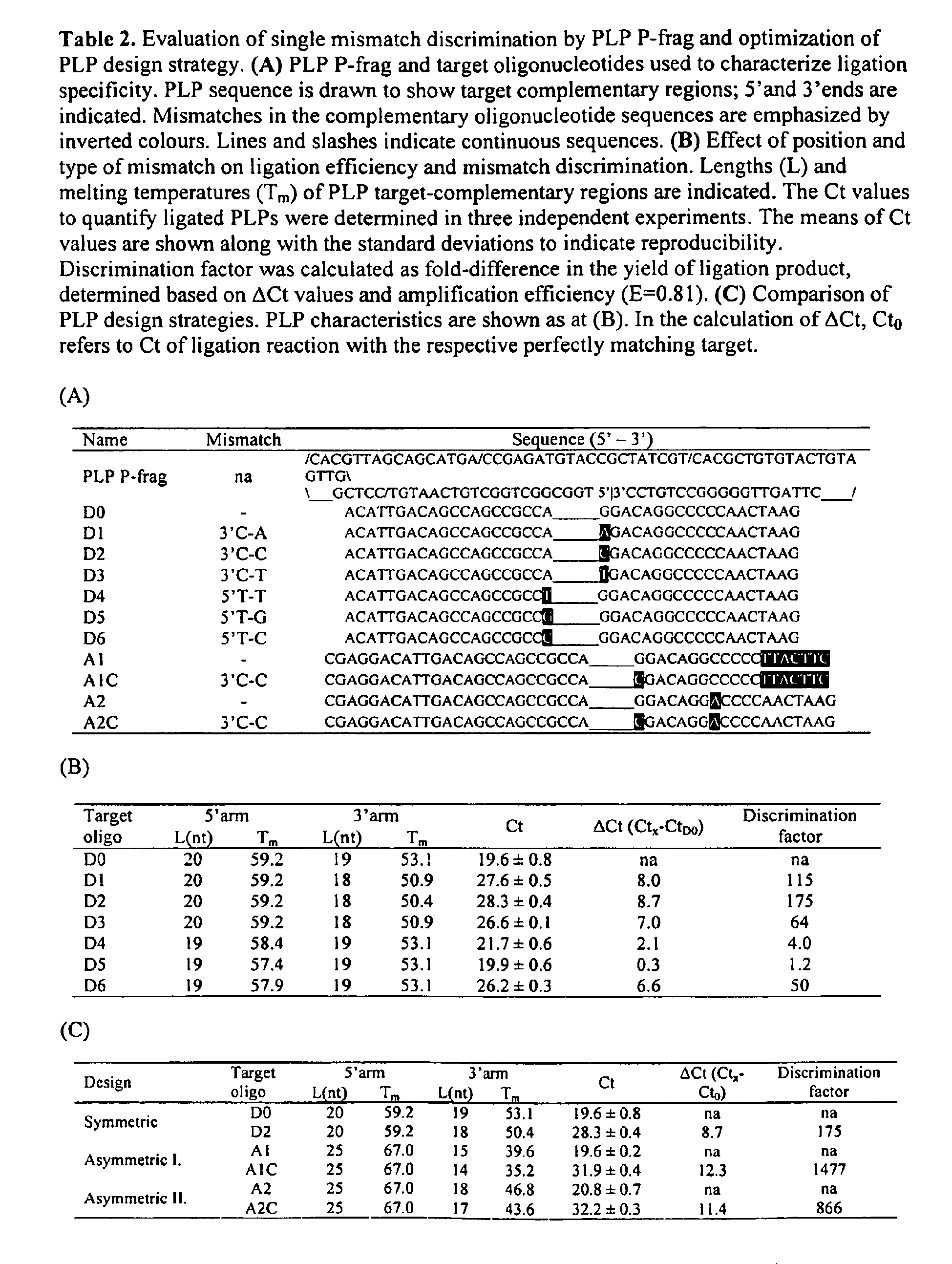

**Table 3. (A) Target-complementary regions and ZipCode sequences of the developed diagnostic PLPs. Nucleotides or gaps due to deletions used to discriminate targets from most similar, non-target sequences are underlined. Gray boxes indicate polymorphism within the target group. (B) Design and experimental characteristics of the PLP set. Probes were named after the targeted species/subgroup. Lengths (L) and melting temperatures (Tₘ) of PLP target-complementary regions are indicated. The number of nucleotides discriminating the targeted sequence from that of the known most similar, non-target organism is shown for each PLP. Sensitivity was defined as the lowest concentration of perfectly matching oligonucleotide that could be detected under standard assay conditions. Discriminatory range gives the magnitude difference between the lowest detectable concentrations of target and of non-target oligonucleotides.**

| (A) | | | | | | | | |
|---|---|---|---|---|---|---|---|---|
| Targeted species/group | 5' target complementary sequence (5'- 3') | | | | | 3'target complementary sequence (5'- 3') | ZipCode sequence (5'-3') | |
| *Phytophthora spp.* | TATCTAGTTAAAAGCAGAGACTTTCGTC | | | | | CTGCTGAAAGTTGC | GTCACGTATGGTTCGCTGCT | |
| *Phytophthora cactorum* | GACTTTCGTCCCCACAGTATAATCAGTATTAAGGAAT | | | | | TATCTAGTTAAAAGCAAG | ACTCCAGTGCCAAGTACGAT | |
| *Verticillium dahliae* | TTTATACCAACGATACTTCTGAGTGTT | | | | | CATCAGTCTCTCTG | CGTTCCTAAAGCTGAGTCTG | |
| *Phytophthora infestans* | TCGATTCGTGGTATGGTTGGCTTCGGCT | | | | | CGTTAATGGAGAAATGC | GCACTAACTGGTCTGGGTCA | |
| *Fusarium oxysporum* | GCGAGTCCCAACACCAAGCTGTGCTTG | | | | | GGAACGCGAATTAAC_ | ATGCAGCGTAGGTATCGACT | |
| *Myrothecium roridum* | CGGTGGTGGCCATGCCGTAAAACACC | | | | | ACTCGCATTGGAGCT | AATGCTCACATCGCAGGTAC | |
| *Phytophthora nicotianae* | TAGTAGTCTTTTTTTCTTTTAAACCCATTCCTTAAT | | | | | GCTTCGGCCTGATT | TCCCGAATGACAAGGCACGA | |
| *Rhizoctonia solani* AG 4-2 | GACTTCTGTCTACTTAATTCATATAAACTCAATT | | | | | CTT CTACTCCCCCTT_ | TGTGATAATTTCGACGAGGC | |
| *Rhizoctonia solani* AG 4-1 | GGTCCAATAAAGTTCCTTCCCCCCTAGAAAA | | | | | AGTCCAA_GGAGAGTA_ | ATTAACTCGACTGCCGCGTG | |
| *Pythium ultimum* | CGAAAAAACGAACGCAACCATGTGAGACACTT | | | | | CGACAGATTCTCGAT | TCGCCGTTGGTCTGTATGCA | |
| *Meloidogyne hapla* | GTTTATCGTTGTGAATGGCTGTCGCTGGTG | | | | | ATTCGAATAGTCTCAAC | CTTCGTGGCTAGTCTGTGAC | |

| (B) | | | | | | | | |
|---|---|---|---|---|---|---|---|---|
| Name | 5'arm | | 3'arm | | Closest non-target relative | # of discr.nt | Sensitivity (fM) | Discriminatory range |
| | Length (nt) | Tₘ (°C) | Length (nt) | Tₘ (°C) | | | | |
| PLP P-spp | 29 | 60.0 | 14 | 39.5 | *Pythium splendens* | 1 | 2 | 10⁵ |
| PLP P-cac | 37 | 65.8 | 18 | 39.6 | *Phytophthora nicotianae* | 2 | 20 | 10⁵ |
| PLP V-dahl | 28 | 58.3 | 14 | 35.4 | *Verticillium alboatrum* | 2 | 2 | 10⁵ |
| PLP P-inf | 28 | 68.9 | 17 | 44.0 | *Phytophthora sojae* | 3 | 2 | 10⁵ |
| PLP F-oxy | 27 | 68.9 | 15 | 41.5 | *Fusarium equiseti* | 3 | 2 | 10⁵ |
| PLP Myr-ror | 27 | 69.0 | 15 | 46.5 | *Myrothecium verrucaria* | 5 | 0.2 | 10⁷ |
| PLP P-nic | 36 | 61.0 | 14 | 43.6 | *Phytophihora cactorum* | 7 | 2 | 10⁶ |
| PLP Rhiz-4-2 | 36 | 59.2 | 15 | 41.0 | *Rhizoctonia solani* AG 4-1 | 7 | 2 | nt |
| PLP Rhiz-4-1 | 32 | 66.2 | 15 | 39.5 | *Rhizoctonia solani* AG 4-2 | 8 | 2 | nt |
| PLP Pyt-u | 32 | 66.2 | 15 | 40.6 | *Pythium splendens* | 10 | 0.2 | nt |
| PLP Mel-h | 30 | 65.7 | 17 | 41.1 | *Meloidogyne incognita* | 15 | 2 | nt |

**Table 4. Pathogen detection using the developed multiplexed PLP set as analyzed on microarrays. For each sample and probe combination the mean signal (± SD) of 9 replicates is shown in the upper row left, where signal was calculated as log₂ (mean Cy3 - local background - assay background). Reliability of the method was evaluated by calculating log₂ (absolute signal/assay background) for each spot, which is called reliability factor. Means (± SD) are shown in the lower row, aligned to right. Criteria for positivity were: mean Cy3 > 2* mean Cy3 local background (cut-off value) and reliability factor > 1. Positive signals are highlighted and shown in bold. (A) Analysis of DNAs of single pathogens. (B) Testing artificial mixtures of pathogen DNAs present in equal ratios. (C) Dynamic range of detection: analysis of DNAs of Myrothecium roridum and Fusarium oxysporum in different ratios.**

| (A) | | | | | | | | | | | |
|---|---|---|---|---|---|---|---|---|---|---|---|
| Samples | Signals corresponding to PLP probes | | | | | | | | | | |
| | P-spp | P-nic | P-cac | P-inf | Pyt-u | Rhiz- 4-1 | Rhiz-4-2 | V-dahl | F-oxy | Myr-ror | Mel-h |
| P. nicotianae, 1 ng | **14_{·}7±0.2** | **13.8±0.2** | 0 | 0 | 0 | 0 | 0 | 0 | 0 | 0 | 0 |
| | **3.9±0.1** | **3.5±0.1** | na | na | na | na | na | na | na | na | na |
| P. cactorum, 1ng | **12.1±03** | 0 | **12.8±0.2** | 0 | 0 | 0 | 0 | 0 | 0 | 0 | 0 |
| | **1.4±0.2** | na | **3.5±0.3** | na | na | na | na | na | na | na | na |
| P. infestans, lng | **14.8±0.3** | 0 | 0 | **12.8±0.2** | 0 | 0 | 0 | 0 | 0 | 0 | 0 |
| | **5.2±0.**1 | na | na | **5.9±0.2** | na | na | na | na | na | na | na |
| P. sojae, 1ng P. sojae, 1ng | **13.9±0.4** | 0 | 0 | 0 | 0 | 0 | 0 | 0 | 0 | 0 | 0 |
| | **8.3±0.2** | na | na | na | na | na | na | na | na | na | na |
| Pyt. ultimum, 1 ng | 0 | 0 | 0 | 0 | **12.8±0.6** | 0 | 0 | 0 | 0 | 0 | 0 |
| | na | na | na | na | **6.1±0.1** | na | na | na | na | na | na |
| R. solani AG 4-1, 1 ng | 0 | 0 | 0 | 0 | 0 | **14.3±0.2** | 0 | 0 | 0 | 0 | 0 |
| | na | na | na | na | na | **3.8±0.1** | na | na | na | na | na |
| R. solani AG 4-21 ng | 0 | 0 | 0 | 0 | 0 | 0 | 0 | 0 | 0 | 0 | 0 |
| | na | na | na | na | na | na | **14.5±0.2 8.3±0.1** | na | na | na | na |
| V. dahliae, 1 ng | 0 | 0 | 0 | 0 | 0 | 0 | 0 | **12.9±1:0.7** | 8.0±0.4 | 0 | 0 |
| | na | na | na | na | na | na | na | **4.6±0.2** | 0.7±0.7 | na | na |
| F. oxysporum 1 ng | 0 | 0 | 0 | 0 | 0 | 0 | 0 | 0 | **15.2±0.2** | 0 | 0 |
| | na | na | na | na | na | na | na | na | **10.8±0.1** | na | na |
| M. roridum, 1 ng | 0 | 0 | 0 | 0 | 0 | 0 | 0 | 0 | 0 | **14.3±0.4** | 0 |
| | na | na | na | na | na | na | na | na | na | **9.3±0.2** | na |
| M. hapla, 1 ng | 0 | 0 | 0 | 0 | 0 | 0 | 0 | 0 | 0 | 0 | **15.0±0.2** |
| | na | na | na | na | na | na | na | na | na | na | **6.5±0.1** |
| V. alboatrum, 1 ng | 10.1±0.5 | 8.7±0.6 | 7.3±0.9 | 7.0±1.0 | 0 | 0 | 8.7±0.5 | 9.0±1.0 | 6.0±0.4 | 7.1±0.7 | 7.5±0.4 |
| | -0.1±0.1 | -3.0±0.6 | 0.3±0.2 | -1.4±0.5 | na | na | -1.1±0.4 | -0.6±0.2 | -1.3±0.9 | 0.1±0.2 | -0.5±0.5 |
| No target (neg. control) | 10.4±0.4 | 9.6±0.4 | 0 | 4.7±1.4 | 0 | 0 | 10.6±0.5 | 8.0±0.5 | 0 | 6.8±0.7 | 9.1±0.8 |
| | -0.1±0.2 | -0.9±0.2 | na | -2.7±1.5 | na | na | 0.1±0.2 | -0.9±0.3 | na | -1.7±0.3 | -0.1±0.3 |

| (B) | | | | | | | | | | | |
|---|---|---|---|---|---|---|---|---|---|---|---|
| Samples | Signals corresponding to PLP probes | | | | | | | | | | |
| | P-spp | P-nic | P-cac | P-inf | Pyt-u | Rhiz-4-1 | Rhiz-4-2 | V-dahl | F-oxy | Myr-ror | Mel-h |
| P. infestans, 500 pg | **11.6±0.4** | 0 | 0 | **11.0±0.4** | 0 | 0 | **12.7±0.6** | 0 | 0 | **12.8±0.5** | 0 |
| R. solani 4-2, 500 pg | | | | | | | | | | | |
| M. roridum , 500 pg | **2.2±0.1** | na | na | **4.6±0.1** | na | na | **5.1±0.1** | na | na | **6.3±0.2** | na |
| P. nicotiane, 500 pg | **13.0±0.4** | **12.3±0.4** | 0 | 0 | **11.9±0.4** | 0 | 0 | 0 | 0 | 0 | **12.0±0.4** |
| Pyt. ultimum, 500 pg | | | | | | | | | | | |
| M. hapla, 500 pg | **3.1±0.1** | **2.4±0.2** | na | na | **6.5±0.6** | na | na | na | na | na | 6.8t0.3 |
| P. cactorum, 500 pg | 8.5±1.2 | 0 | **11.8±0.2** | 0 | 0 | **13.5±0.4** | 0 | **12.4±0.2** | 0 | 0 | 0 |
| R. solani 4-1, 500 pg | | | | | | | | | | | |
| V. dahliae, 500 pg | -2.6±1.4 | na | 6.3±0.2 | na | na | **3.3±0.1** | na | **5.1±0.1** | na | na | na |

| (C) | | | | | | | | | | | |
|---|---|---|---|---|---|---|---|---|---|---|---|
| Samples | Signals corresponding to PLP probes | | | | | | | | | | |
| | P-spp | P-nic | P-cac | P-inf | Pyt-u | Rhiz-4-1 | Rhiz-4-2 | V-dahl | F-oxy | Myr-ror | Mel-h |
| M. roridum, 500 pg | 0 | 0 | 0 | 0 | 0 | 0 | 0 | 0 | **11.2±0.7** | **14.1±0.7** | 0 |
| F. oxysporum, 500 pg | na | na | na | na | na | na | na | na | **9.0±1.1** | **7.0±03** | na |
| M. roridum, 500 pg | 0 | 0 | 0 | 0 | 0 | 0 | 0 | 0 | **10.6±0.4** | **13.8±0.7** | |
| F. oxysporum, 50 pg | na | na | na | na | na | na | na | na | **8.0±0.7** | **75±0.3** | na |
| M. roridum, 500 pg | 0 | 0 | 0 | 0 | 0 | 0 | 0 | 0 | **8.7±0.3** | **12.7±0.4** | 0 |
| F. oxysporum, 5 pg | na | na | na | na | na | na | na | na | **S.It0.4** | **7.8±0.2** | na |
| M. roridum, 500 pg | 0 | 0 | 0 | 0 | 0 | 0 | 0 | 0 | **9.0±0.3** | **13.9±0.6** | 0 |
| F. oxysporum, 0.5 pg | na | na | na | na | na | na | na | na | **1 .5+0.2** | **8.0±0.3** | na |
| M. roridum, 5 pg | 0 | 0 | 0 | 0 | 0 | 0 | 0 | 0 | **14.±0.7** | **9.6±0.3** | 0 |
| F. oxysporum, 500 pg | na | na | na | na | na | na | na | na | **10.3±0.2** | **1.2±0.1** | na |
| M. roridum, 0.5 pg | 0 | 0 | 0 | 0 | 0 | 0 | 0 | 0 | **14.5±1.0** | 5.9±0.9 | 0 |
| F. oxysporum, 500 pg | na | na | na | na | na | na | na | na | **10.0±0.4** | -1.5±0.5 | na |

**Table 5: The targeted groups of pathogens and the most similar, non-target micro-organisms for each designed PRI-lock probe. The number of discriminating nucleotides are indicated.**

| **PRI-Lock** | **Target** | **Closest non-target relative** | **Discrimination (nt)** |
|---|---|---|---|
| PRI Phyt | *Phytophthora* spp. | *Pythium splendens* | 1 |
| PRI P.inf | *Phytophihora infestans* | *Phytophthora sojae* | 3 |
| PRI M.ror | *Myrothecium roridum* | *Myrothecium verrucaria* | 5 |

**Table 6: Ct values of real-time PCR reactions performed on ligation mixtures containing different PRI-lock probe and target combinations.**

| Template | PRI-Locks | Primers Phyt._fw, Phyt rv | Primers P.inf_fw, P.inf_rv | Primers M.ror-fw, 10 M.ror_rv |
|---|---|---|---|---|
| *Phytophthora infestans* | PRI_Phyt | 24.07 | 40.00 | 40.00 |
| *Phytophthora infestans* | PRI_P.inf | 40.00 | 22.49 | 40.00 |
| *Myrothecium roridum* | PRI_M.ror | 40.00 | 40.00 | 22.04 |
| *Phytophthora infestans* | PRI_Phyt | | | |
| | PRI_P.inf | 24.46 | 21.97 | 40.00 |
| | PRI_M.ror | | | |
| *Myrothecium roridum* | PRI_Phyt | | | |
| | PRI_P.inf | 40.00 | 40.00 | 21.59 |
| | PRI_M.ror | | | |
| *Phytophthora infestans*: *Myrothecium roridum* | RPI_Phyt | | | |
| | PRI_P.inf | 25.10 | 22.77 | 21.46 |
| | PRI_M.ror | | | |
| *No target* | PRI_Phyt | | | |
| | PRI_P.inf | 40.00 | 40.00 | 40.00 |
| | PRI_M.ror | | | |

## Claims

1. A standard padlock nucleotide probe comprising from 5' to 3':
a) a target specific nucleotide sequence 1 (T1)
b) a generic reverse primer binding site
c) a nucleotide sequence bearing desthio-biotine
d) a linker of at least 12 nucleotides
e) a unique cleavable sequence
f) a generic forward primer binding site
g) a unique ZIP-code sequence
h) a target specific nucleotide sequence 2 (T2),
wherein the T1 and T2 sequences are designed to be complementary to adjacent nucleotide stretches on the same target in such a way that after hybridization (and ligation of the outer ends) the padlock probe forms a circular molecule.

2. A padlock probe according to claim 1, wherein the unique cleavable sequence is a poly-uracil sequence.

3. A padlock probe according to claim 2, wherein the poly-uracil sequence functions as a first member of a binding pair.

4. A padlock probe according to any of claims 1-3, **characterised in that** it also comprises a T7 RNA polymerase recognition site.

5. A padlock probe according to claim 4, wherein the T7 RNA polymerase recognition site is located 5' of the generic forward primer binding site.

6. A padlock probe according to any of claims 1-5, wherein the ZIP code is the complementary strand of a nucleotide sequence on an array or other element

7. A padlock nucleotide probe (PRI lock) comprising from 5' to 3'
a) a target specific nucleotide sequence 1 (T1)
b) a unique reverse primer binding site
c) a nucleotide sequence bearing desthio-biotine
d) a linker of at least 12 nucleotides
e) a unique cleavable sequence
f) a unique forward primer binding site
g) a target specific nucleotide sequence 2 (T2),
wherein the T1 and T2 sequences are designed to be complementary to adjacent nucleotide stretches on the same target in such a way that after hybridization (and ligation of the outer ends) the padlock probe forms a circular molecule.

8. Padlock probe according to claim 7, wherein the unique cleavable sequence is a poly-uracil sequence.

9. Padlock probe according to claim 7 or 8 which comprises a universal ZIP code, situated between the unique forward primer binding site and the target specific nucleotide sequence 2 (T2).

10. Padlock probe according to any of claims 1-9, wherein the poly-uracil sequence comprises from 2-100, preferably 2-50, more preferably 2-255, most preferably 3-10 nucleotides.

11. Padlock probe according to any of claims 1-10, wherein the first target specific sequence (T1) has a length of about 10 to about 75 nucleotides, preferably of about 20 to about 50 nucleotides and most preferably of about 25 to about 40 nucleotides.

12. Padlock probe according to any of claims 1-11, wherein the second target specific sequence (T2) has a length of about 10 to about 30 nucleotides, preferably of about 10 to about 20 nucleotides.

13. A method for the detection of a target nucleotide sequence comprising:
a. adding to a sample which contains said target, one or more padlock nucleotide probes according to any of claims 1-6, which have target specific sequences capable of hybridisation to said target;
b. allowing annealing of the padlock nucleotide probe to said target;
c. ligating the padlock nucleotide probe to itself;
d. capturing the padlock nucleotide probes via the desthio-biotine by bringing them into contact with a solid support coated with streptavidine
e. linearising the padlock nucleotide probe;
f. washing of the solid support to remove any unbound oligonucleotides
g. elution of the PLP probe from the solid support
h. detection of the ZIP-code.

14. Method according to claim 13, wherein the detection of the ZIP-code comprises the steps or:
i. amplification of the padlock nucleotide probe using generic primers;
j. labelling the amplified padlock nucleotide probe;
k. testing for presence of the ZIP-code by hybridising said padlock nucleotide probe with at least one sequence which is capable of hybridisation with said ZIP-code, wherein said hybridisation preferably takes place at a solid support, such as a (micro-) array.

15. Method according to claim 13, wherein detection of the ZIP-code comprises the steps of:
i. testing for presence of the ZIP-code by hybridising said padlock nucleotide probe with at least one sequence which is capable of hybridisation with said ZIP-code, wherein said hybridisation preferably takes place at a solid support, such as a (micro-) array or gold bead; and
j. labelling of the hybridized padlock nucleotide probe on the solid support with a fluorescent probe directed against the first member of a binding pair of the padlock probe or with fluorescently labelled Biobarcode labelled gold beads.

16. A method for the detection of a target nucleotide sequence comprising:
a. adding to a sample which contains said target, one or more padlock nucleotide probes according to claims 7-12, which have target specific sequences capable of hybridisation to said target;
b. allowing annealing of the padlock nucleotide probe to said target;
c. circularisation of padlock probe by ligation;
d. capturing the padlock nucleotide probe via the desthio-biotine using a solid support coated with streptavidine;
e. linearising the padlock nucleotide probe;
f. eluting the padlock nucleotide probe from the solid support,
g. amplifying the eluted padlock nucleotide probe using unique primers;
h. monitoring and/or detection of amplification.

17. Method according to claim 16, wherein monitoring and/or detection of amplification is performed using an intercalating dye, preferably ethidiumbromide or SYBR Green.

18. Method according to claim 17, wherein monitoring and/or detection of amplification is performed using a universal probe, preferably a TaqMan probe.

19. Method according to any of claims 13-18, wherein the unique cleavage sequence is a polyuracil sequence and linearization is achieved by treatment with uracil_N-glycosidase and endonuclease IV.

20. Method according to any of claims 13-19, wherein biotin is used to elute the probe from the solid support.

21. Method according to any of claims 13-20, wherein a denaturation step with NaOH is performed before capturing the padlock nucleotide probes.

22. Method according to any of claims 13-21, wherein the solid support is a bead or a column.

23. Use of a padlock probe according to any of claims 1-10 for the multiplex detection of nucleotide sequences.

24. Kit comprising multiple padlock probes according to any of claims 1-10, wherein each padlock probe is designed to recognise a unique target.

## Patentansprüche

1. Standard-Padlocknucleotidsonde, umfassend von 5' nach 3':
a) eine zielspezifische Nucleotidsequenz 1 (T1),
b) eine generische Rückwärtsprimer-Bindestelle;
c) eine Nucleotidsequenz, die Desthio-Biotin trägt,
d) einen Linker von mindestens 12 Nucleotiden,
e) eine einzigartige spaltbare Sequenz,
f) eine generische Vorwärtsprimer-Bindestelle,
g) eine einzigartige ZIP-Code-Sequenz,
h) eine zielspezifische Nucleotidsequenz 2 (T2),
wobei die T1- und T2-Sequenzen so entworfen sind, dass sie komplementär zu angrenzenden Nucleotidabschnitten auf dem gleichen Ziel sind, und zwar dergestalt, dass die Padlocksonde nach Hybridisierung (und Ligierung der äußeren Enden) ein ringförmiges Molekül bildet.

2. Padlocksonde nach Anspruch 1, wobei die einzigartige spaltbare Sequenz eine Poly-Uracil-Sequenz ist.

3. Padlocksonde nach Anspruch 2, wobei die Poly-Uracil-Sequenz als ein erstes Mitglied eines Bindepaares fungiert.

4. Padlocksonde nach einem der Ansprüche 1 bis 3, **dadurch gekennzeichnet, dass** sie auch eine T7-RNA-Polymerase-Erkennungsstelle umfasst.

5. Padlocksonde nach Anspruch 4, wobei die T7-RNA-Polymerase-Erkennungsstelle sich 5' zur generischen Vorwärtsprimer-Bindestelle befindet.

6. Padlocksonde nach einem der Ansprüche 1 bis 5, wobei der ZIP-Code der komplementäre Strang einer Nucleotidsequenz auf einem Array oder einem anderen Element ist.

7. Padlocknucleotidsonde (PRI-lock), umfassend von 5' nach 3'
a) eine zielspezifische Nucleotidsequenz 1 (T1),
b) eine einzigartige Rückwärtsprimer-Bindestelle,
c) eine Nucleotidsequenz, die Desthio-Biotin trägt,
d) ein Linker von mindestens 12 Nucleotiden,
e) eine einzigartige spaltbare Sequenz,
f) eine einzigartige Vorwärtsprimer-Bindestelle,
g) eine zielspezifische Nucleotidsequenz 2 (T2),
wobei die T1- und T2-Sequenzen so entworfen sind, dass sie komplementär zu angrenzenden Nucleotidabschnitten auf dem gleichen Ziel sind, und zwar dergestalt, dass die Padlocksonde nach Hybridisierung (und Ligierung der äußeren Enden) ein ringförmiges Molekül bildet.

8. Padlocksonde nach Anspruch 7, wobei die einzigartige spaltbare Sequenz eine Poly-Uracil-Sequenz ist.

9. Padlocksonde nach Anspruch 7 oder 8, welche einen universellen ZIP-Code umfasst, der zwischen der einzigartigen Vorwärtsprimer-Bindestelle und der zielspezifischen Nucleotidsequenz 2 (T2) gelegen ist.

10. Padlocksonde nach einem der Ansprüche 1 bis 9, wobei die Poly-Uracil-Sequenz 2 -100, bevorzugt 2 - 50, mehr bevorzugt 2 - 255 und am meisten bevorzugt 3-10 Nucleotide umfasst.

11. Padlocksonde nach einem der Ansprüche 1 bis 10, wobei die erste zielspezifische Sequenz 1 (T1) eine Länge von etwa 10 bis etwa 75 Nucleotiden, vorzugsweise von etwa 20 bis etwa 50 Nucleotide, und am meisten bevorzugt von etwa 25 bis etwa 40 Nucleotiden hat.

12. Padlocksonde nach einem der Ansprüche 1 bis 11, wobei die zweite zielspezifische Nucleotidsequenz 2 (T2) eine Länge von etwa 10 bis etwa 30 Nucleotiden, vorzugsweise von etwa 10 bis etwa 20 Nucleotiden hat.

13. Verfahren zum Nachweis einer Zielnucleotidsequenz, umfassend:
a. Hinzufügen zu einer Probe, welche das Ziel enthält, einer oder mehrerer Padlocknucleotidsonden nach einem der Ansprüche 1- 6, welche zielspezifische Sequenzen haben, die zur Hybridisierung an das Ziel fähig sind;
b. Ermöglichen des Annealings der Padlocknucleotidsonde an das Ziel;
c. Ligieren der Padlocknucleotidsonde mit sich selbst;
d. Einfangen der Padlocknucleotidsonden über das Desthio-Biotin durch Inkontaktbringen dieser mit einem festen Träger, der mit Strepatvidin beschichtet ist,
e. Linearisieren der Padlocknucleotidsonde;
f. Waschen des festen Trägers, um jedwede ungebundenen Oligonucleotide zu entfernen,
g. Eluieren der PLP-Sonde von dem festen Träger,
h. Nachweis des ZIP-Codes.

14. Verfahren nach Anspruch 13, wobei der Nachweis des ZIP-Codes die Schritte umfasst:
i. Amplifikation der Padlocknucleotidsonde unter Verwendung von generischen Primern;
j. Markieren der amplifizierten Padlocknucleotidsonde;
k. Prüfen auf das Vorhandensein des ZIP-Codes durch Hybridisierung der Padlocknucleotidsonde mit mindestens einer Sequenz, welche zur Hybridisierung mit dem ZIP-Code fähig ist, wobei die Hybridisierung vorzugsweise auf einem festen Träger wie einem (Micro-)Array stattfindet.

15. Verfahren nach Anspruch 13, wobei der Nachweis des ZIP-Codes die Schritte umfasst:
i. Prüfen auf das Vorhandensein des ZIP-Codes durch Hybridisierung der Padlocknucleotidsonde mit mindestens einer Sequenz, welche zur Hybridisierung mit dem ZIP-Code fähig ist, wobei die Hybridisierung vorzugsweise auf einem festen Träger wie einem (Micro-)Array oder einer Goldperle stattfindet; und
j. Markieren der hybridisierten Padlocknucleotidsonde auf dem festen Träger mit einer fluoreszierenden Sonde, die gegen das erste Mitglied eines Bindepaares der Padlocknucleotidsonde gerichtet ist, oder mit fluoreszenzmarkierten Biobarcode-markierten Goldperlen.

16. Verfahren zum Nachweis einer Zielnucleotidsequenz, umfassend:
a. Hinzufügen zu einer Probe, welche das Ziel enthält, einer oder mehrerer Padlocknucleotidsonden nach einem der Ansprüche 7 - 12, welche zielspezifische Sequenzen haben, die zur Hybridisierung an das Ziel fähig sind;
b. Ermöglichen des Annealings der Padlocknucleotidsonde an das Ziel;
c. Zirkularisieren der Padlocknucleotidsonde durch Ligierung;
d. Einfangen der Padlocknucleotidsonde über das Desthio-Biotin unter Verwendung eines festen Trägers, der mit Strepatvidin beschichtet ist;
e. Linearisieren der Padlocknucleotidsonde;
f. Eluieren der Padlocknucleotidsonde von dem festen Träger,
g. Amplifizieren der eluierten Padlocknucleotidsonde unter Verwendung von einzigartigen Primern;
h. Beobachten und/oder Nachweis der Amplification.

17. Verfahren nach Anspruch 16, wobei das Beobachten und/oder der Nachweis der Amplifikation unter Verwendung eines interkalierenden Farbstoffes, vorzugsweise Ethidiumbromid oder SYBR Green, durchgeführt wird.

18. Verfahren nach Anspruch 17, wobei das Beobachten und/oder der Nachweis der Amplifikation unter Verwendung einer universellen Sonde, vorzugsweise einer TaqMan-Sonde, durchgeführt wird.

19. Verfahren nach einem der Ansprüche 13 - 18, wobei die einzigartige Spaltsequenz eine Poly-Uracil-Sequenz ist und die Linearisierung durch Behandlung mit Uracil-N-Glycosidase und Endonuclease IV erreicht wird.

20. Verfahren nach einem der Ansprüche 13 - 19, wobei Biotin zur Eluierung der Sonde von dem festen Träger verwendet wird.

21. Verfahren nach einem der Ansprüche 13 - 20, wobei ein Denaturierungsschritt mit NaOH vor dem Einfangen der Padlocknucleotidsonden durchgeführt wird.

22. Verfahren nach einem der Ansprüche 13 - 21, wobei der feste Träger eine Perle oder eine Säule ist.

23. Verwendung einer Padlocksonde nach einem der Ansprüche 1 - 10 zum vielfachen Nachweis von Nucleotidsequenzen.

24. Kit umfassend vielfache Padlocksonden nach einem der Ansprüche 1 - 10, wobei jede Padlocksonde so entworfen ist, dass sie ein einzigartiges Ziel erkennt.

## Revendications

1. Sonde nucléotidique cadenas standard, comprenant de 5' à 3' :
a) une séquence nucléotidique cible spécifique 1 (T1),
b) un site de liaison d'amorce inverse générique,
c) une séquence nucléotidique portant de la déthiobiotine,
d) un segment de liaison d'au moins 12 nucléotides,
e) une séquence clivable unique,
f) un site de liaison d'amorce directe générique,
g) une séquence d'adressage unique,
h) une séquence nucléotidique cible spécifique 2 (T2),
dans laquelle les séquences T1 et T2 sont conçues pour être complémentaires de segments nucléotidiques adjacents sur la même cible de telle manière que, après hybridation (et soudure des extrémités extérieures), la sonde cadenas forme une molécule circulaire.

2. Sonde cadenas selon la revendication 1, dans laquelle la séquence clivable unique est une séquence polyuracile.

3. Sonde cadenas selon la revendication 2, dans laquelle la séquence polyuracile fait fonction de premier membre d'une paire de liaison.

4. Sonde cadenas selon l'une quelconque des revendications 1 à 3, **caractérisée en ce qu'**elle comprend également un site de reconnaissance de l'ARN polymérase de T7.

5. Sonde cadenas selon la revendication 4, dans laquelle le site de reconnaissance de l'ARN polymérase de T7 est situé en 5' par rapport au site de liaison d'amorce directe générique.

6. Sonde cadenas selon l'une quelconque des revendications 1 à 5, dans laquelle la séquence d'adressage est le brin complémentaire d'une séquence nucléotidique sur un réseau ou un autre élément.

7. Sonde nucléotidique cadenas (sonde PRI lock) comprenant de 5' à 3' :
a) une séquence nucléotidique cible spécifique 1 (T1),
b) un site de liaison d'amorce inverse unique,
c) une séquence nucléotidique portant de la déthiobiotine,
d) un segment de liaison d'au moins 12 nucléotides,
e) une séquence clivable unique,
f) un site de liaison d'amorce directe unique,
g) une séquence nucléotidique cible spécifique 2 (T2),
dans laquelle les séquences T1 et T2 sont conçues pour être complémentaires de segments nucléotidiques adjacents sur la même cible de telle manière que, après hybridation (et soudure des extrémités extérieures), la sonde cadenas forme une molécule circulaire.

8. Sonde cadenas selon la revendication 7, dans laquelle la séquence clivable unique est une séquence polyuracile.

9. Sonde cadenas selon la revendication 7 ou la revendication 8, qui comprend une séquence d'adressage universelle située entre le site de liaison d'amorce directe unique et la séquence nucléotidique cible spécifique 2 (T2).

10. Sonde cadenas selon l'une quelconque des revendications 1 à 9, dans laquelle la séquence polyuracile comprend 2 à 100, de préférence 2 à 50, plus préférentiellement 2 à 255 et de la façon la plus préférentielle 3 à 10 nucléotides.

11. Sonde cadenas selon l'une quelconque des revendications 1 à 10, dans laquelle la première séquence cible spécifique (T1) a une longueur d'environ 10 à environ 75 nucléotides, de préférence d'environ 20 à environ 50 nucléotides et de la façon la plus préférentielle d'environ 25 à environ 40 nucléotides.

12. Sonde cadenas selon l'une quelconque des revendications 1 à 11, dans laquelle la deuxième séquence cible spécifique (T2) a une longueur d'environ 10 à environ 30 nucléotides, de préférence d'environ 10 à environ 20 nucléotides.

13. Procédé de détection d'une séquence nucléotidique cible, comprenant les étapes consistant à :
a) ajouter à un échantillon qui contient ladite cible une ou plusieurs sondes nucléotidiques cadenas selon l'une quelconque des revendications 1 à 6 possédant des séquences cibles spécifiques capables de s'hybrider à ladite cible ;
b) permettre à la sonde nucléotidique cadenas de se circulariser avec ladite cible ;
c) souder la sonde nucléotidique cadenas à elle-même ;
d) capturer les sondes nucléotidiques cadenas par l'intermédiaire de la déthiobiotine en les mettant en contact avec un support solide revêtu de streptavidine ;
e) linéariser la sonde nucléotidique cadenas ;
f) laver le support solide afin d'éliminer les éventuels oligonucléotides non liés ;
g) éluer la sonde cadenas du support solide ;
h) détecter la séquence d'adressage.

14. Procédé selon la revendication 13, dans lequel la détection de la séquence d'adressage comprend les étapes consistant à :
i) amplifier la sonde nucléotidique cadenas en utilisant des amorces génériques ;
j) marquer la sonde nucléotidique cadenas amplifiée ;
k) rechercher la présence de la séquence d'adressage en hybridant ladite sonde nucléotidique cadenas avec au moins une séquence qui est capable de s'hybrider avec ladite séquence d'adressage, ladite hybridation se faisant de préférence sur un support solide tel qu'un (micro-) réseau.

15. Procédé selon la revendication 13, dans lequel la détection de la séquence d'adressage comprend les étapes consistant à :
i) rechercher la présence de la séquence d'adressage en hybridant ladite sonde nucléotidique cadenas avec au moins une séquence qui est capable de s'hybrider avec ladite séquence d'adressage, ladite hybridation se faisant de préférence sur un support solide tel qu'un (micro-) réseau ou une bille d'or ; et
j) marquer la sonde nucléotidique cadenas hybridée sur le support solide avec une sonde fluorescente dirigée contre le premier membre d'une paire de liaison de la sonde cadenas ou avec des billes d'or dotées d'un marquage fluorescent au moyen d'un marqueur Biobarcode.

16. Procédé de détection d'une séquence nucléotidique cible, comprenant les étapes consistant à :
a) ajouter à un échantillon qui contient ladite cible une ou plusieurs sondes nucléotidiques cadenas selon les revendications 7 à 12 possédant des séquences cibles spécifiques capables de s'hybrider à ladite cible ;
b) permettre à la sonde nucléotidique cadenas de se circulariser avec ladite cible ;
c) circulariser la sonde cadenas par soudure ;
d) capturer la sonde nucléotidique cadenas par l'intermédiaire de la déthiobiotine en utilisant un support solide revêtu de streptavidine ;
e) linéariser la sonde nucléotidique cadenas ;
f) éluer la sonde nucléotidique cadenas du support solide ;
g) amplifier la sonde nucléotidique cadenas éluée en utilisant des amorces uniques ;
h) monitorer et/ou détecter l'amplification.

17. Procédé selon la revendication 16, dans lequel le monitorage et/ou la détection de l'amplification se font en utilisant un colorant intercalant, de préférence le bromure d'éthidium ou le vert SYBR.

18. Procédé selon la revendication 17, dans lequel le monitorage et/ou la détection de l'amplification se font en utilisant une sonde universelle, de préférence une sonde TaqMan.

19. Procédé selon l'une quelconque des revendications 13 à 18, dans lequel la séquence clivable unique est une séquence polyuracile et la linéarisation se fait par traitement avec de l'uracile N-glycosidase et une endonucléase IV.

20. Procédé selon l'une quelconque des revendications 13 à 19, dans lequel de la biotine est utilisée pour éluer la sonde du support solide.

21. Procédé selon l'une quelconque des revendications 13 à 20, dans lequel une étape de dénaturation au NaOH est réalisée avant la capture des sondes nucléotidiques cadenas.

22. Procédé selon l'une quelconque des revendications 13 à 21, dans lequel le support solide est une bille ou une colonne.

23. Utilisation d'une sonde cadenas selon l'une quelconque des revendications 1 à 10 pour la détection multiplex de séquences nucléotidiques.

24. Kit comprenant de multiples sondes cadenas selon l'une quelconque des revendications 1 à 10, chaque sonde cadenas étant conçue pour reconnaître une cible unique.
